# EUROPEAN PATENT SPECIFICATION

(11) **EP 1 987 066 B1**
(45) Date of publication and mention of the grant of the patent: **29.12.2010**
(21) Application number: 07704693.6
(22) Date of filing: 21.02.2007
(51) Int. Cl.: C07K 16/28, A61K 39/395, A61P 25/02

(54) **METHODS FOR TREATING DEMYELINATING DISEASES**
VERFAHREN ZUR BEHANDLUNG VON DEMYELINIERENDEN KRANKHEITEN
PROCÉDÉS DE TRAITEMENT DES MALADIES DÉMYÉLINISANTES

(30) Priority: 22.02.2006 EP 06110301; 18.04.2006 US 792795 P
(43) Date of publication of application: 05.11.2008
(73) Proprietor: University of Zürich, 8006 Zürich (CH)
(72) Inventor: BECHER, Burkhard, CH-8124 Maur (CH)
(74) Representative: Merck Serono SA - Geneva Intellectual Property
(86) International application number: PCT/EP2007/051692
(87) International publication number: WO 2007/096396

(56) References cited:
- EP-A- 1 004 312
- WO-A-02/32374
- WO-A-03/008452
- WO-A-03/059387
- WO-A-2005/011605
- WO-A-2005/019258
- WO-A-2006/009114
- K. KINOSHITA ET AL: "Blockade if IL-18 receptor signaling delays the onset of autoimmune disease in MRL-Faslpr mice" THE JOURNAL OF IMMUNOLOGY, vol. 173, no. 8, 2004, pages 5312-5318, XP002381999 2004
- B. SERGI AND I. PENTTILA: "Interleukin 18 receptor" J BIOL REGUL HOMEOST AGENTS, vol. 18, 2004, pages 55-61, XP002382000
- M. NOBUMOTO ET AL: "Overproduction and simple purification of mitochondrial adenylate kinase isozymes" J. BIOCHEM. MIL. BIOL. AND BIOPHYSIC., vol. 2, 1998, pages 13-20, XP008064518
- R and D systems: "anti human IL-18Ralpha antibody", MAB840
- R and D systems: "Anti human IL-18R alpha/IL-1 R5 antibodies", MAB840
- Diaclone catalog number 854.900.000: "CDw218a antibody",
- Ebiosciences Catalog number 14-7183: "Affinity purified anti-human IL-18 Receptor alpha chain",

### SEQUENCE LISTING

<110> University of Zurich
<120> METHODS FOR TREATING AUTOIMMUNE OR DEMYELINATING DISEASES
<130> EXT108 EP EPA
<160> 12
<170> PatentIn version 3.3
<210> 1
   <211> 1626
   <212> DNA
   <213> Homo sapiens
<400> 1
   atgaattgta gagaattacc cttgaccctt tgggtgctta tatctgtaag cactgcagaa 60
   tcttgtactt cacgtcccca cattactgtg gttgaagggg aacctttcta tctgaaacat 120
   tgctcgtgtt cacttgcaca tgagattgaa acaaccacca aaagctggta caaaagcagt 180
   ggatcacagg aacatgtgga gctgaaccca aggagttcct cgagaattgc tttgcatgat 240
   tgtgttttgg agttttggcc agttgagttg aatgacacag gatcttactt tttccaaatg 300
   aaaaattata ctcagaaatg gaaattaaat gtcatcagaa gaaataaaca cagctgtttc 360
   actgaaagac aagtaactag taaaattgtg gaagttaaaa aatttttcca gataacctgt 420
   gaaaacagtt actatcaaac actggtcaac agcacatcat tgtataagaa ctgtaaaaag 480
   ctactactgg agaacaataa aaacccaacg ataaagaaga acgccgagtt tgaagatcag 540
   gggtattact cctgcgtgca tttccttcat cataatggaa aactatttaa tatcaccaaa 600
   accttcaata taacaatagt ggaagatcgc agtaatatag ttccggttct tcttggacca 660
   aagcttaacc atgttgcagt ggaattagga aaaaacgtaa ggctcaactg ctctgctttg 720
   ctgaatgaag aggatgtaat ttattggatg ttcggggaag aaaatggatc ggatcctaat 780
   atacatgaag agaaagaaat gagaattatg actccagaag gcaaatggca tgcttcaaaa 840
   gtattgagaa ttgaaaatat tggtgaaagc aatctaaatg ttttatataa ttgcactgtg 900
   gccagcacgg gaggcacaga caccaaaagc ttcatcttgg tgagaaaagc agacatggct 960
   gatatcccag gccacgtctt cacaagagga atgatcatag ctgttttgat cttggtggca 1020
   gtagtgtgcc tagtgactgt gtgtgtcatt tatagagttg acttggttct attttataga 1080
   catttaacga gaagagatga aacattaaca gatggaaaaa catatgatgc ttttgtgtct 1140
   tacctaaaag aatgccgacc tgaaaatgga gaggagcaca cctttgctgt ggagattttg 1200
   cccagggtgt tggagaaaca ttttgggtat aagttatgca tatttgaaag ggatgtagtg 1260
   cctggaggag ctgttgttga tgaaatccac tcactgatag agaaaagccg aagactaatc 1320
   attgtcctaa gtaaaagtta tatgtctaat gaggtcaggt atgaacttga aagtggactc 1380
   catgaagcat tggtggaaag aaaaattaaa ataatcttaa ttgaatttac acctgttact 1440
   gacttcacat tcttgcccca atcactaaag cttttgaaat ctcacagagt tctgaagtgg 1500
   aaggccgata aatctctttc ttataactca aggttctgga agaaccttct ttacttaatg 1560
   cctgcaaaaa cagtcaagcc aggtagagac gaaccggaag tcttgcctgt tctttccgag 1620
   tcttaa 1626
<210> 2
   <211> 541
   <212> PRT
   <213> Homo sapiens
<400> 2
   Met Asn Cys Arg Glu Leu Pro Leu Thr Leu Trp Val Leu Ile Ser Val
   1               5                   10                  15
   Ser Thr Ala Glu Ser Cys Thr Ser Arg Pro His Ile Thr Val Val Glu
               20                  25                  30
   Gly Glu Pro Phe Tyr Leu Lys His Cys Ser Cys Ser Leu Ala His Glu
           35                  40                  45
   Ile Glu Thr Thr Thr Lys Ser Trp Tyr Lys Ser Ser Gly Ser Gln Glu
       50                  55                  60
   His Val Glu Leu Asn Pro Arg Ser Ser Ser Arg Ile Ala Leu His Asp
   65                  70                  75                  80
   Cys Val Leu Glu Phe Trp Pro Val Glu Leu Asn Asp Thr Gly Ser Tyr
                   85                  90                  95
   Phe Phe Gln Met Lys Asn Tyr Thr Gln Lys Trp Lys Leu Asn Val Ile
               100                 105                 110
   Arg Arg Asn Lys His Ser Cys Phe Thr Glu Arg Gln Val Thr Ser Lys
           115                 120                 125
   Ile Val Glu Val Lys Lys Phe Phe Gln Ile Thr Cys Glu Asn Ser Tyr
       130                 135                 140
   Tyr Gln Thr Leu Val Asn Ser Thr Ser Leu Tyr Lys Asn Cys Lys Lys
   145                 150                 155                 160
   Leu Leu Leu Glu Asn Asn Lys Asn Pro Thr Ile Lys Lys Asn Ala Glu
                   165                 170                 175
   Phe Glu Asp Gln Gly Tyr Tyr Ser Cys Val His Phe Leu His His Asn
               180                 185                 190
   Gly Lys Leu Phe Asn Ile Thr Lys Thr Phe Asn Ile Thr Ile Val Glu
           195                 200                 205
   Asp Arg Ser Asn Ile Val Pro Val Leu Leu Gly Pro Lys Leu Asn His
       210                 215                 220
   Val Ala Val Glu Leu Gly Lys Asn Val Arg Leu Asn Cys Ser Ala Leu
   225                 230                 235                 240
   Leu Asn Glu Glu Asp Val Ile Tyr Trp Met Phe Gly Glu Glu Asn Gly
                   245                 250                 255
   Ser Asp Pro Asn Ile His Glu Glu Lys Glu Met Arg Ile Met Thr Pro
               260                 265                 270
   Glu Gly Lys Trp His Ala Ser Lys Val Leu Arg Ile Glu Asn Ile Gly
           275                 280                 285
   Glu Ser Asn Leu Asn Val Leu Tyr Asn Cys Thr Val Ala Ser Thr Gly
       290                 295                 300
   Gly Thr Asp Thr Lys Ser Phe Ile Leu Val Arg Lys Ala Asp Met Ala
   305                 310                 315                 320
   Asp Ile Pro Gly His Val Phe Thr Arg Gly Met Ile Ile Ala Val Leu
                   325                 330                 335
   Ile Leu Val Ala Val Val Cys Leu Val Thr Val Cys Val Ile Tyr Arg
               340                 345                 350
   Val Asp Leu Val Leu Phe Tyr Arg His Leu Thr Arg Arg Asp Glu Thr
           355                 360                 365
   Leu Thr Asp Gly Lys Thr Tyr Asp Ala Phe Val Ser Tyr Leu Lys Glu
       370                 375                 380
   Cys Arg Pro Glu Asn Gly Glu Glu His Thr Phe Ala Val Glu Ile Leu
   385                 390                 395                 400
   Pro Arg Val Leu Glu Lys His Phe Gly Tyr Lys Leu Cys Ile Phe Glu
                   405                 410                 415
   Arg Asp Val Val Pro Gly Gly Ala Val Val Asp Glu Ile His Ser Leu
               420                 425                 430
   Ile Glu Lys Ser Arg Arg Leu Ile Ile Val Leu Ser Lys Ser Tyr Met
           435                 440                 445
   Ser Asn Glu Val Arg Tyr Glu Leu Glu Ser Gly Leu His Glu Ala Leu
       450                 455                 460
   Val Glu Arg Lys Ile Lys Ile Ile Leu Ile Glu Phe Thr Pro Val Thr
   465                 470                 475                 480
   Asp Phe Thr Phe Leu Pro Gln Ser Leu Lys Leu Leu Lys Ser His Arg
                   485                 490                 495
   Val Leu Lys Trp Lys Ala Asp Lys Ser Leu Ser Tyr Asn Ser Arg Phe
   500 505 510
   Trp Lys Asn Leu Leu Tyr Leu Met Pro Ala Lys Thr Val Lys Pro Gly
   515 520 525
   Arg Asp Glu Pro Glu Val Leu Pro Val Leu Ser Glu Ser
   530 535 540
<210> 3
   <211> 119
   <212> PRT
   <213> Artificial
<220>
   <223> VH
<400> 3
   Glu Val Gln Leu Gln Gln Ser Gly Ala Glu Leu Val Lys Pro Gly Ala
   1 5 10 15
   Ser Val Lys Leu Ser Cys Thr Thr Ser Gly Phe Asn Ile Lys Asp Ile
   20 25 30
   Tyr Ile Tyr Trp Val Lys Gln Arg Pro Glu Gln Gly Leu Glu Trp Val
   35 40 45
   Gly Arg Ile Asp Pro Ala Asn Gly Asp Thr Lys Tyr Gly Pro Asn Phe
   50 55 60
   Gln Asp Lys Ala Thr Ile Thr Ala Asp Thr Ser Ser Asn Thr Ala Tyr
   65 70 75 80
   Leu Gln Leu Arg Ser Leu Thr Ser Glu Asp Thr Ala Val Tyr Tyr Cys
   85 90 95
   Ala Arg Arg Gly Asn Tyr Gly Ala Gly Phe Gly Tyr Trp Gly Gln Gly
   100 105 110
   Thr Leu Val Thr Val Ser Ala
   115
<210> 4
   <211> 108
   <212> PRT
   <213> Artificial
<220>
   <223> VL
<400> 4
   Asp Ile Gln Met Thr Gln Ser Pro Ala Ser Leu Ser Ala Ser Val Gly
   1 5 10 15
   Glu Thr Val Thr Ile Thr Cys Arg Ala Ser Gly Asn Ile His Asn Tyr
   20 25 30
   Leu Ala Trp Tyr Gln Gln Arg Gln Gly Lys Ser Pro Gln Ile Leu Val
   35 40 45
   Tyr Asn Ala Lys Thr Leu Ala Asp Gly Val Ser Ser Arg Phe Ser Gly
   50 55 60
   Ser Gly Ser Gly Thr Gln Tyr Ser Leu Asn Ile Asn Ser Leu Gln Pro
   65 70 75 80
   Glu Asp Phe Gly Thr Tyr Phe Cys Gln His Phe Trp Ser Thr Pro Tyr
   85 90 95
   Thr Phe Gly Gly Gly Thr Lys Leu Glu Ile Lys Arg
   100 105
<210> 5
   <211> 10
   <212> PRT
   <213> Artificial
<220>
   <223> CDR1 Heavy
<400> 5
   Gly Phe Asn Ile Lys Asp Ile Tyr Ile Tyr
   1 5 10
<210> 6
   <211> 18
   <212> PRT
   <213> Artificial
<220>
   <223> CDR2 Heavy
<400> 6
   Arg Ile Asp Pro Ala Asn Gly Asp Thr Lys Tyr Gly Pro Asn Phe Gln
   1 5 10 15
   Asp Lys
<210> 7
   <211> 10
   <212> PRT
   <213> Artificial
<220>
   <223> CDR3 Heavy
<400> 7
   Arg Gly Asn Tyr Gly Ala Gly Phe Gly Tyr
   1 5 10
<210> 8
   <211> 11
   <212> PRT
   <213> Artificial
<220>
   <223> CDR1 Light
<400> 8
   Arg Ala Ser Gly Asn Ile His Asn Tyr Leu Ala
   1 5 10
<210> 9
   <211> 7
   <212> PRT
   <213> Artificial
<220>
   <223> CDR2 Light
<400> 9
   Asn Ala Lys Thr Leu Ala Asp
   1 5
<210> 10
   <211> 9
   <212> PRT
   <213> Artificial
<220>
   <223> CDR3 Light
<400> 10
   Gln His Phe Trp Ser Thr Pro Tyr Thr
   1 5
<210> 11
   <211> 21
   <212> PRT
   <213> Artificial
<220>
   <223> MOG 35-55
<400> 11
   Met Glu Val Gly Trp Tyr Arg Ser Pro Phe Ser Arg Val Val His Leu
   1 5 10 15
   Tyr Arg Asn Gly Lys
   20
<210> 12
   <211> 17
   <212> PRT
   <213> Artificial
<220>
   <223> SMARTA p11
<400> 12
   Gly Pro Asp Ile Tyr Lys Gly Val Tyr Gln Phe Lys Ser Val Glu Phe
   1 5 10 15
   Asp

## Description

The present invention relates to novel therapeutic or prophylactic treatment in human subjects. The present invention results in part from the discovery that antagonists of IL-18Rα are effective *in vivo* for treating diseases.

Accordingly, the invention provides a method of treating, preventing or ameliorating the symptoms of a demyelinating disease (such as multiple sclerosis) in a subject, preferably a human subject, said method comprising administering to the subject a therapeutically effective amount of an antagonist of IL-18Ra. The invention also pertains to the use of an antagonist of IL-18Rα in the manufacture of a medicament for the treatment of a demyelinating diseases (such as multiple sclerosis).

### BACKGROUND

Demyelinating diseases are a group of pathologies that involve abnormalities in myelin sheaths of the nervous system. Many congenital metabolic disorders affect the developing myelin sheath, mainly in the CNS, and demyelination is a feature of many neurological disorders.

The most known chronic inflammatory demyelinating disease of the central nervous system in humans is multiple sclerosis. The onset of multiple sclerosis (MS) typically occurs during ages 20 to 40. Women are affected approximately twice as often as men. Over time, MS may result in the accumulation of various neurological disabilities. Clinical disability in MS is presumed to be a result of repeated inflammatory injury with subsequent loss of myelin and axons, leading to tissue atrophy.

MS is manifested in physical symptoms (relapses and disability progression), central nervous system (CNS) inflammation, brain atrophy and cognitive impairment. Presenting symptoms include focal sensory deficits, focal weakness, visual problems, imbalance and fatigue. Sexual impairment and sphincter dysfunction may occur. Approximately half of the patients with MS may experience cognitive impairment or depression.

MS is now considered to be a multi-phasic disease, and periods of clinical quiescence (remissions) occur between exacerbations. Remissions vary in length and may last several years but are infrequently permanent.

Four courses of the disease are individualized: relapsing-remitting (RR), secondary progressive (SP), primary progressive (PP) and progressive relapsing (PR) multiple sclerosis. More than 80% of patients with MS initially display a RR course with clinical exacerbation of neurological symptoms, followed by a recovery that may or may not be complete (Lublin and Reingold, Neurology, 1996, 46:907-911).

During RRMS, accumulation of disability results from incomplete recovery from relapses. Approximately, half of the patients with RRMS switch to a progressive course, called SPMS, 10 years after the diseased onset. During the SP phase, worsening of disability results from the accumulation of residual symptoms after exarcerbation but also from insidious progression between exacerbations (*Lublin and Reingold above*). 10% of MS patients have PPMS which is characterized by insidious progression of the symptoms from the disease onset. Less than 5 % of patients have PRMS and are often considered to have the same prognosis as PPMS. It is suggested that distinct pathogenic mechanisms may be involved in different patient sub-groups and have wide-ranging implications for disease classification (Lassmann et al., 2001, Trends Mol. Med., 7, 115-121*;* Lucchinetti et al., Curr. Opin. Neurol., 2001, 14, 259-269*).*

MS onset is defined by the occurrence of the first neurological symptoms of CNS dysfunction. Advances in cerebrospinal fluid (CSF) analysis and magnetic resonance imaging (MRI) have simplified the diagnostic process and facilitated early diagnostic *(*Noseworthy et al., The New England Journal of Medicine, 2000, 343, 13, 938-952). The International Panel on the Diagnosis of MS issued revised criteria facilitating the diagnosis of MS and including MRI together with clinical and para-clinical diagnostic methods (Mc Donald et al., 2001, Ann. Neurol., 50:121-127).

Documents WO 2006/009114 and WO 02/32374 deal with antibody against IL-18Rα, and envisage the treatment of demyelinating disease with these antibodies, among other diseases. Documents WO 03/008452 and B. Sergi et al, "Interleukin 18 receptor", Journal of Biological regulators and homeostatic agents 2004, 18: 35-61 have contradictory results which do not incite to go on for the treatment of demyelinating diseases with antibodies against IL-18Rα.

Treatments currently available for the treatment of multiple sclerosis essentially act against the symptoms of the disease. Consequently, there is a strong need for alternative therapies that provide improved clinical benefits to patients.

### SUMMARY OF THE PRESENT INVENTION

The present invention relates to novel therapeutic or prophylactic treatment in human subjects. The present invention results in part from the discovery that antagonists of IL-18Rα are effective *in vivo* for treating diseases.

Accordingly, the invention provides a method of treating, preventing or ameliorating the symptoms of a demyelinating disease in a subject, preferably a human subject, said method comprising administering to the subject a therapeutically effective amount of an antagonist of IL-18Rα. In a particular aspect, the demyelinating disease is multiple sclerosis. The invention also pertains to the use of an antagonist of IL-18Rα in the manufacture of a medicament for the treatment of a demyelinating disease. In a particular aspect, the demyelinating disease is multiple sclerosis.

In a particular aspect, the invention resides in the method or use as defined above wherein the subject is affected by relapsing remitting (RR) multiple sclerosis, secondary progressive (SP) multiple sclerosis, primary progressive (PP) multiple sclerosis or progressive relapsing (PR) multiple sclerosis.

In a particular aspect, the antagonist is an antibody that selectively binds to IL18-Rα, in particular to the extra-cellular domain of IL18-Rα. More specifically the antibody selectively binds to the polypeptide of SEQ ID NO: 2, and even more specifically, the antibody selectively binds to residues 1-329 of SEQ ID NO: 2, or residues 19-329 of SEQ ID NO: 2, or residues 330 to 350 of SEQ ID NO: 2 or residues 351 to 541 of SEQ ID NO: 2. In a particular aspect, the antibody selectively binds to residues 19-219 of SEQ ID N: 2, or residues 122-329 of SEQ ID N: 2. In another particular aspect, the antibody selectively binds to residues 19-132 of SEQ ID N: 2, or residues 122-219 of SEQ ID N: 2, or residues 213-329 of SEQ ID N: 2.

Another object of the present invention resides in the method or use as defined above wherein the antibody selectively binds to an epitope located in the extracellular domain of human IL-18Rα. In a particular aspect, the epitope is located into the amino acids 19 to 329 of SEQ ID NO: 2, or into the amino acid residues 19 to 219 of SEQ ID NO: 2, or into amino acid residues 122 to 329 of SEQ ID NO: 2. In another particular aspect, the epitope is located into the amino acids 19-132 of SEQ ID N: 2, or 122-219 of SEQ ID N: 2, or 213-329 of SEQ ID N: 2.

A further object of this invention resides in the method or use as defined above wherein the antibody have an antigen binding domain that comprises six CDRs having an amino acid sequence selected from the group consisting of: SEQ ID NO: 5, SEQ ID NO: 6, SEQ ID NO: 7, SEQ ID NO: 8, SEQ ID NO: 9 and SEQ ID NO: 10. In a particular aspect, the antibody comprises a VH domain comprising an amino acid sequence of SEQ ID NO: 3 and a VL domain comprising an amino acid sequence of SEQ ID NO: 4. More specifically, the antibody comprises a VH domain consisting in an amino acid sequence of SEQ ID NO: 3 and a VL domain consisting in an amino acid sequence of SEQ ID NO: 4. Even more specifically, the antibody comprises a human IgG1 as Ig constant heavy region; an Ig constant light region selected from the group consisting of a human Ig kappa constant domain and a human Ig lambda constant domain; an Ig variable heavy region having an amino acid sequence of SEQ ID NO : 3; and an Ig variable light region having an amino acid sequence of SEQ ID NO : 4.

In a very specific aspect, the present invention resides in the method or use as defined above wherein the antibody is selected form the group consisting of Monoclonal Anti-human IL-18Rα clone 70614, monoclonal Anti-human IL-18Rα clone 70625, monoclonal anti-human IL-18Rα clone B-E43 and monoclonal anti-human IL-18Rα clone H44.

The invention further resides in the method or use as defined above wherein the antibody is an antibody that compete with Ab1, Monoclonal Anti-human IL-18Rα clone 70614, monoclonal Anti-human IL-18Rα clone 70625, monoclonal anti-human IL-18Rα clone B-E43 and/or monoclonal anti-human IL-18Rα clone H44 for binding to human 1L-18Rα.

The invention also relates to the method or use as defined above wherein the antibody is obtainable by the process comprising the step of
- immunizing a host animal with an immunizing agent comprising residues 19-329 of SEQ ID NO: 2, or residues 19-219 of SEQ ID N: 2, or residues 122-329 of SEQ ID N: 2, or residues 19-132 of SEQ ID N: 2, or residues 122-219 of SEQ ID N: 2, or residues 213-329 of SEQ ID N: 2.
- fusing the lymphocytes produced by said host animal with an immortalized cell line to form hybridoma cells,
- selecting clones of hybridoma cells producing antibodies directed against the immunizing peptide,
- testing the activity of the antibodies produced by the different clones of hybridoma cells in an EAE animal model and selecting an antibody that inhibit the progression of EAE in said animal model,
- producing the monoclonal antibody secreted.
More specifically, the immunizing agent may consists of residues 19-329 of SEQ ID NO: 2, or residues 19-219 of SEQ ID N: 2, or residues 122-329 of SEQ ID N: 2, or residues 19-132 of SEQ ID N: 2, or residues 122-219 of SEQ ID N: 2, or residues 213-329 of SEQ ID N: 2 or a fusion protein thereof.

The invention further resides in the method or use as defined above wherein the antibody is a human antibody, a humanized antibody or a fragment thereof.

The invention further resides in the method or use as defined above wherein the antagonist inhibits the activity of IL18Rα in Antigen presenting cells. In a particular aspect, the antigen presenting cells are selected from the group consisting of monomorphonucleated phagocytes, polymorphonucleated phagocytes, dendritic cells and Natural Killer cells.

The invention further resides in the method or use as defined above wherein the inhibition of IL-18Rα leads to the decrease of IL-17 producing Helper T cells.

The invention also relates to the method or use as defined above wherein the antagonist of IL-18Rα is administered in conjunction with a second therapeutic agent for treating or preventing MS. In a particular aspect, the antagonist of IL-18Rα is administered in conjunction with corticosteroids, immunosuppressive drugs, neuro-protective agents, immunomodulatory drugs or interferons.

Other aspects of this invention include a product comprising an antagonist of IL-18Ra and a corticosteroid, an immunosuppressive drug, a neuro-protective agent, an immunomodulatory drug or an interferon as a combined preparation for simultaneous, separate or sequential use in the therapy of MS in a mammalian subject, preferably a human subject. An antagonist of IL-18Rα for use as a medicament. And an antibody that selectively binds to IL18-Rα, in particular to the extra-cellular domain of IL18-Rα, for use as a medicament.

### LEGEND TO THE FIGURES

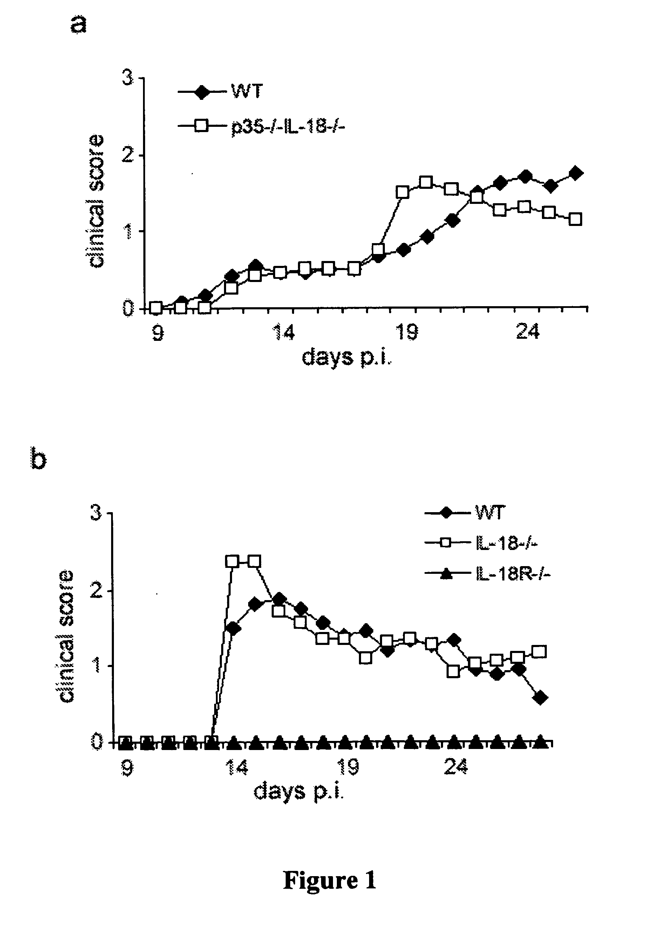

Figure 1 : IL-18R signaling, independent of IL-18, is required for EAE induction. Mice were actively immunized with MOG₃₅₋₅₅ in CFA and injected with pertussis toxin i.p. on days 0 and 2. (a) EAE progression in p35_{-/-}xIL-18_{-/-} double knockout and wt mice. Shown is one representative of 2 experiments (*n* = 5 mice/group).
   (b) EAE progression in wt, IL-18_{-/-} and IL-18Rα_{-/-} mice. Shown is one representative of 3 experiments (*n* . 5 mice/group).

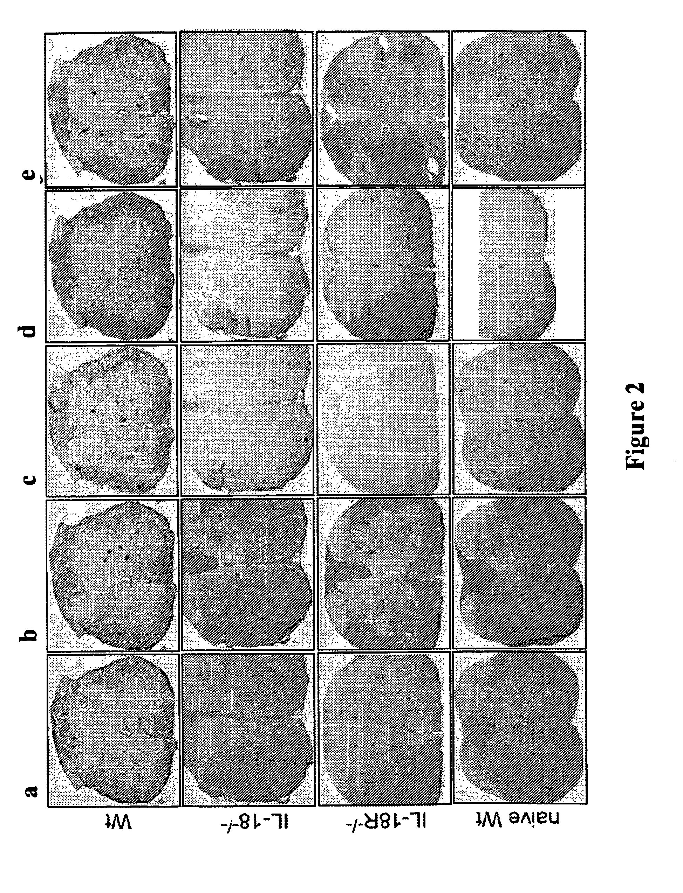

Figure 2 : IL-18R signaling, independent of IL-18, is required for EAE induction. Mice were actively immunized with MOG₃₅₋₃₅ in CFA and injected with pertussis toxin i.p. on days 0 and 2. (a) H&E, (b) LFB, (c) CD3, (d) MAC3 and (e) B220 stainings of PFA-fixed spinal cords from wt (score 2), IL-18-/- (score 2), IL-18Rα-/- (score 0) EAE mice and a naïve mouse showing infiltration relative to disease score.

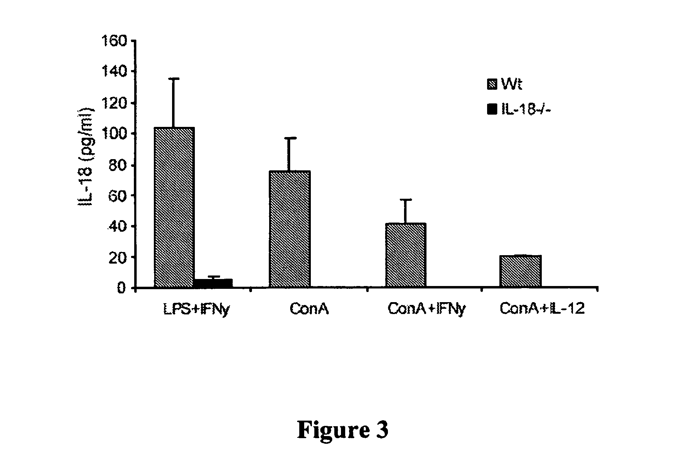

Figure 3 : IL-18-/- LN cells do not produce IL-18 in agreement with their proposed genotype. ELISA assessing IL-18 secretion by naïve wt and IL-18-/- LN cells, stimulated for 16 hours with the indicated mixes of 1 µg/ml LPS, 100 Units/ml IFNγ, 5 µg/ml Concanavalin A (ConA) and 2.5 ng/ml IL-12.

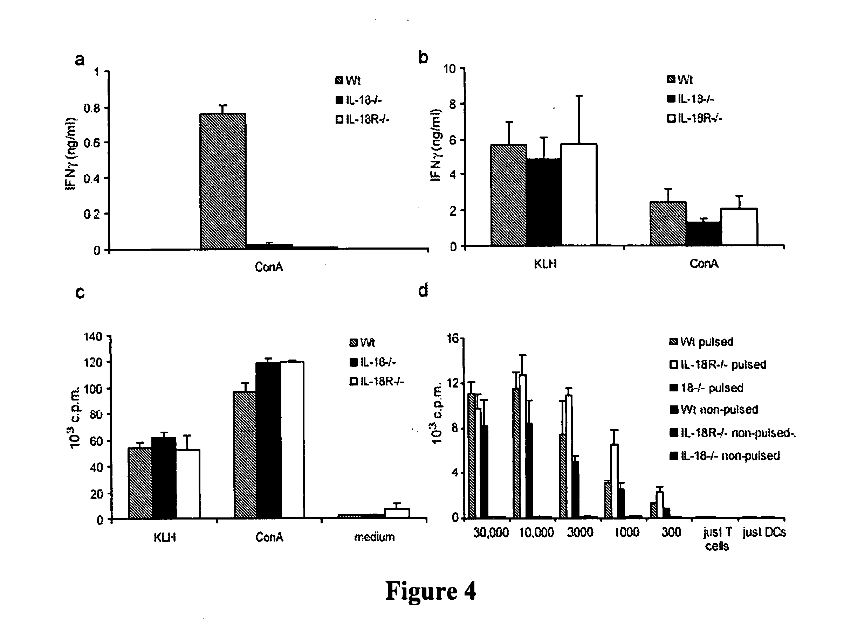

Figure 4 : IL-18 and IL-18Rα are required for mitogen-stimulated T cell activation but not for Th1 development. (a) ELISA assessing IFNγ secretion by naïve wt, IL-18_{-/-} and IL-18Rα_{-/-}LN cells, stimulated for 16 hours with 5 µg/ml Concanavalin A (ConA). (b,c) Mice were immunized with 200 µg KLH and 7 days later LNs were isolated and restimulated.
   (b) ELISA of IFNγ in supernatant from KLH immunized mice restimulated in duplicate with 50 µg/ml KLH or 5 µg/ml ConA for 48 hours.
   (c) Proliferation assay of LN cells from KLH immunized mice restimulated in triplicate with 50 µg/ml KLH, 5 µg/ml ConA or medium for 48 hours. ³H-thymidine was added to the culture 24 hours prior to measuring proliferation in counts per minute (CPM).
   (d) BM-derived DC's were generated from wt, IL-18_{-/-} and IL18R_{-/-} mice, matured with LPS and subsequently pulsed with 1 µg/ml SMARTA peptide, p11. p11-specific CD4+ T cells were obtained from naïve SMARTA-Tg mice and cocultured with the peptide-pulsed, irradiated (2000 rads) DC's for 72h when proliferation was assessed by thymidine incorporation in counts per minute (CPM).

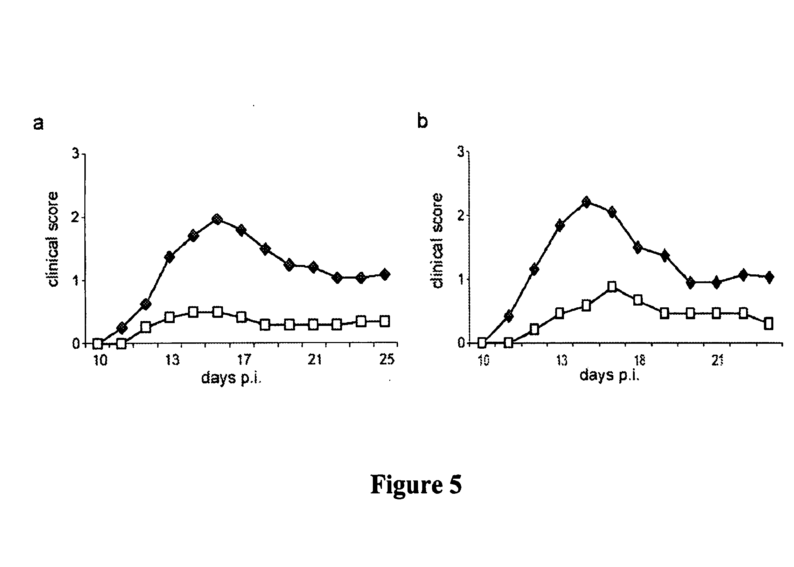

Figure 5 : An alternative IL-18Rα-binding ligand induces EAE in IL-18_{-/-} mice. (a) IL-18_{-/-} mice were treated with 450 µg anti-IL-18Rα antibody (white square) or control IgG (black rhomb) 1 day pre-immunization with MOG₃₅₋₅₅ and with 300 µg antibody for every 3 days thereafter. Shown is one representative of 2 experiments (n = 5 mice/group).
   (b) IL-18_{-/-} mice (n = 6 mice/group) were immunized with MOG₃₅₋₅₅ and treated with 300 µg anti-IL-18Rα antibody (white square) or control IgG (black rhomb) at the first sign of disease.

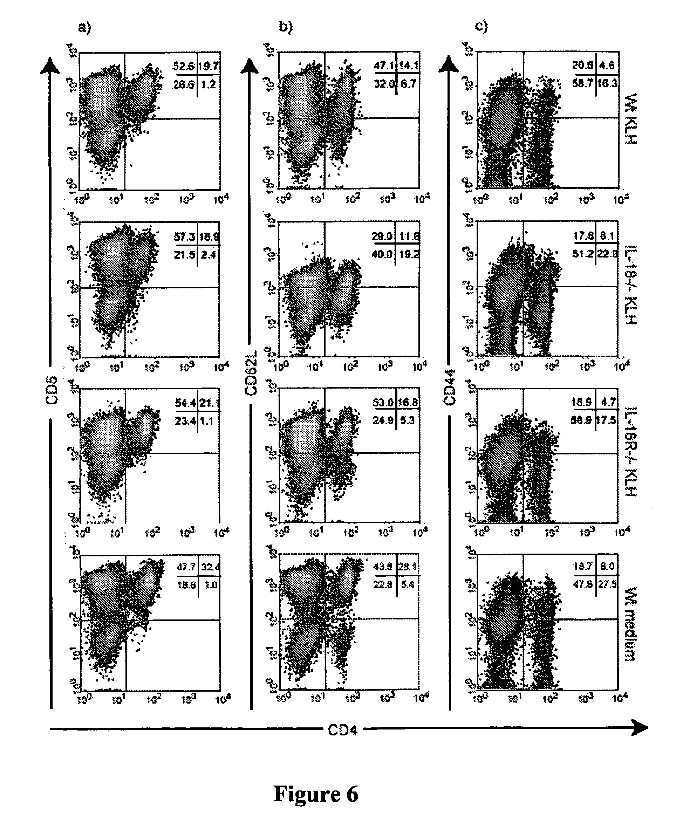

Figure 6 : IL-18R-/- CD4+ T cells are activated similar to wt and IL-18-/- CD4+ T cells. FACS of splenocytes derived from KLH immunized wt, IL-18-/- and IL-18R-/- mice, restimulated *in vitro* for 2 days with 50 µg/ml KLH or medium. After 2 days, spleen cells were stained with CD4-FITC and (a) CD5-APC, (b) CD62L-bio-SA-PerCP-Cy5.5 or (c) CD44-PE.

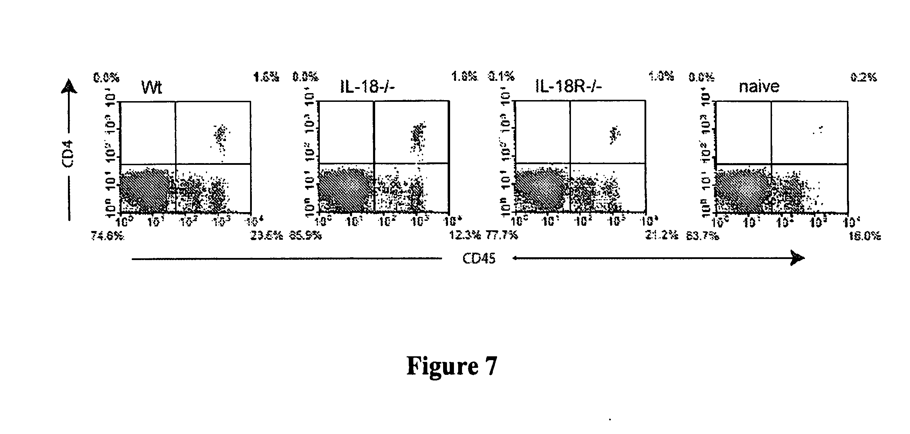

Figure 7 : IL-18Rα_{-/-}CD4+T cells infiltrate the CNS to the same extent as wt and IL-18_{-/-} CD4+ T cells prior to disease onset. wt, IL-18_{-/-} and IL-18Rα_{-/-} mice were actively immunized with MOG₃₅₋₅₅ and on day 7 post-immunization mice were perfused with PBS and the CNS was isolated. A gradient was performed to isolate microglia cells and the infiltration of inflammatory cells in this portion was assessed by flow cytometry. Cells were stained with CD45-PerCP and CD4-APC. IL-18Rα_{-/-} CD4+ T cells invade the CNS and do so to the same as wt and IL-18_{-/-}CD4+ T cells on day 7 post-immunization.

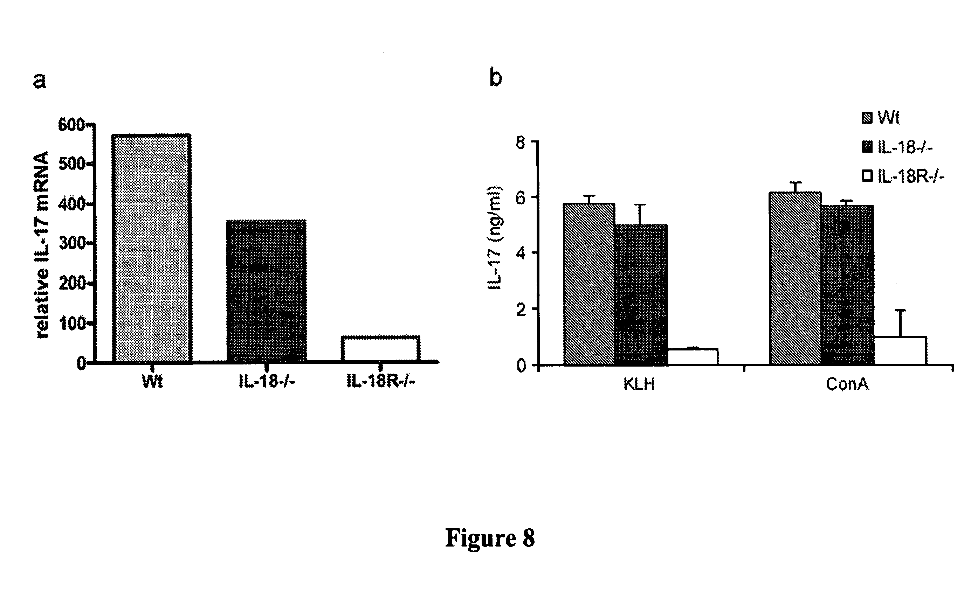

Figure 8 : The IL-18Rα lesion affects the production of IL-17 and the development of THIL-17 cells. Wt, IL-184_{-/-} and IL-18Rα_{-/-} mice were immunized with KLH and 7 days later, splenocytes were isolated and restimulated with 50 µg/ml KLH. (a) Real-time PCR comparison of IL-17 mRNA expression by wt, IL-18_{-/-} and IL-18Rα_{-/-} lymphocytes after 2 days *in vitro* restimulation with KLH. Results are normalized to β-actin expression and analyzed in duplicate. (b) ELISA of IL-17 protein expression by lymphocytes restimulated for 2 days with KLH *in vitro* in duplicate. Data combine at least 2 mice per group.

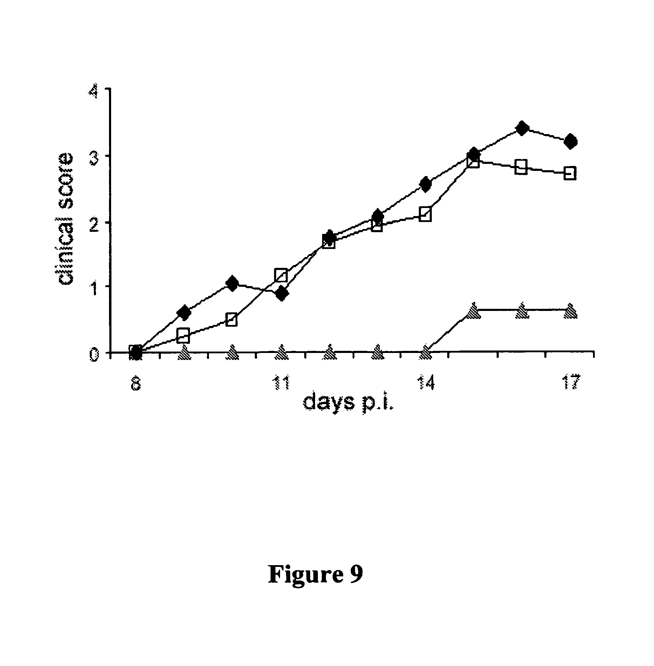

Figure 9 : The absence of IL-18Rα does not lesion T cells or B cells. BM-chimeric mice were generated by transferring 12-25 x10⁶ BM-cells into lethally irradiated wt mice. 6 weeks later, reconstituted IL-18Rα_{-/-}→wt (grey triangle), IL-18Rα_{-/-} + RAG_{-/-}→wt (white square) and wt→wt (black rhomb) bone-marrow chimeric mice were actively immunized with MOG₃₅₋₅₅ peptide and clinical score was assessed. The presence of IL-18Rα on non-T and -B cells derived from the RAG_{-/-} bone marrow rescued the susceptibility of IL-18Rα_{-/-}→wt mice to EAE.

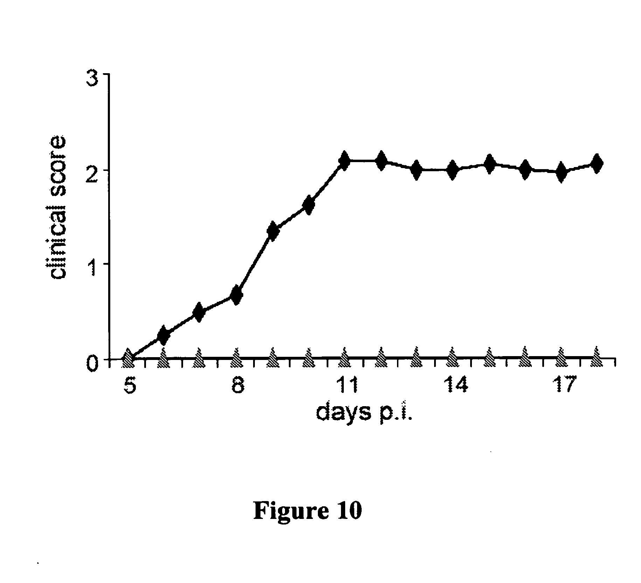

Figure 10 : IL-18Rα_{-/-} mice are resistant to the passive transfer of EAE. MOG-reactive lymphocytes were generated by actively immunizing wt mice, isolating spleen and LN cells after 11 days and restimulating them for 4 days *in vitro* with 20 µg/ml MOG₃₅₋₅₅ and 2.5 ng/ml IL-12. EAE was induced in recipient mice by the adoptive transfer of 20-30x10⁶ MOG-reactive lymphocytes into IL-18Rα_{-/-} (grey triangle) and wt (black rhomb) mice. Shown is one representative of 2 experiments (n = 5 mice/group).

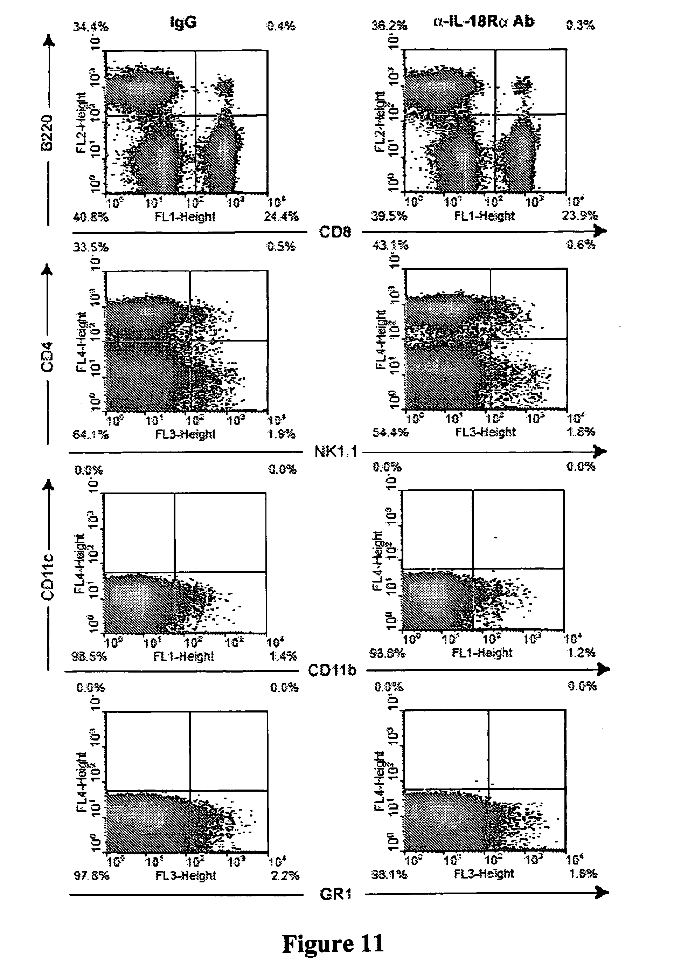

Figure 11 : Anti-IL-18Rα Ab treatment does not alter the composition of peripheral immune cells. IL-18_{-/-} mice were treated with 300 µg anti-IL-18Rα antibody or control IgG 1 day pro-immunization with MOG₃₅₋₅₅. 7 days later, spleens were isolated, homogenized and immune cell composition was assessed by flow cytometry. Cells were stained for CD8-FITC, CD4-APC, NK1.1-bio-SA-PerCP and B220-PE or CD11b-FITC, CD11c-APC and GR1-bio-SA-PerCP. There is no difference in immune cell composition in anti-IL-18Rα Ab-treated IL-18-/- mice. Shown is one representative FACS of 2 mice/group.

### DETAILED DESCRIPTION OF THE INVENTION

The present invention is based in part on the discovery that treating animals with agents that antagonize IL-18Rα reduces symptoms in an animal model for MS. Accordingly, the invention provides methods of treating, preventing or ameliorating the symptoms of a demyelinating diseases (such as MS) in a human subject by administering a therapeutically effective amount of an antagonist of IL-18Rα to the subject.

IL-18 Receptor has been described as a heterodimer consisting of a ligand-binding IL-18Rα-subunit (also named IL-1Rrp or IL-1 R5 in the literature) and a signaling IL-18Rβ-subunit. Downstream signaling of the IL-18R, like that of the TLR pathway, activates IRAK4 and MyD88. IL-18Rα is expressed on lymphocytes and has more recently been found to be expressed on accessory cells (Kaser,A. et al. Blood 103, 648-655 (2004), Tomura,M. et al. Immunol. 160, 3759-3765 (1998), Xu,D. et al. J. Exp. Med. 188, 1485-1492 (1998), Yoshimoto,T. et al. J. Immunol. 161, 3400-3407 (1998)).

While it is established that IL-18 can bind to the IL-18R complex, its affinity to IL-18Rα alone is only weak (Boraschi,D. et al. Eur. Cytokine Netw. 9, 205-212 (1998), Torigoe,K. et aL J. Biol. Chem. 272, 25737-25742 (1997)). IL-18 collaborates with IL-12 to stimulate the production of IFN-γ by T cells and can independently stimulate the cytotoxic activity of NK cells. IL-18 and IL-12 act synergistically to polarize T cells towards a TH1 cytokine response, which was thought to be a prerequisite for encephalitogenicity.

IL-18_{-/-} mice have been described as being EAE resistant and insufficient NK-cell activation in IL-18_{-/-} mice was thought to be the cause for the inability to generate an encephalitogenic immune response (Shi,F.D., et al., J. Immunol. 165, 3099-3104 (2000)). Nevertheless, the proposed role of IL-18 in EAE causes a dilemma given the clearly protective activity of IL-12 (Cua,D.J. et al. Nature 421, 744-748 (2003), Becher,B., et al., J. Clin. Invest 110, 493-497 (2002)).

The inventor now demonstrates that, in contrast to the previously published data, IL-18 does not exert a visible pathogenic effect in EAE as deduced by the susceptibility of IL-18_{-/-} mice to EAE. However, deletion of its proposed receptor (IL-18Rα) results in complete resistance to EAE induction, suggesting the presence of an alternative ligand (IL-18RL) with encephalitogenic properties. As the affinity of IL-18 to IL-18Rα is fairly poor and requires heterotrimerization with IL-18Rβ for increased affinity, the possibility that there is another ligand with higher affinity for IL-18Rα is very strong. There are a number of orphan receptors within the IL-1R superfamily and given the fact that these receptor subunits form heterodimers with one another, it is most likely that the IL-18Rα not only has different binding partners, but also different ligands.

The inventor demonstrates here the potency of this putative ligand by significantly attenuating disease development in IL-18_{-/-} mice using anti-IL-18Rα antibodies. Given that the accepted IL-18Rα-ligand, IL-18, was not present in these mice and that their cellular constituents were not affected as a result of injecting the antibody, these results provide substantial evidence for the existence of such an alternative IL-18Rα ligand.

Despite the importance of T cells during EAE, the inventor shows here that deletion of IL-18Rα does not affect T cell priming with regards to expansion and Th1 polarization. Alternatively, IL-18 and IL-18Rα are both required for efficient T cell activation when stimulated with the mitogen ConA, which concurs with the finding that IL-18_{-/-} mice have a defect in stimulating IFNγ secretion, as observed in various bacterial and viral infectious models. In agreement with a lack of disturbance at the level of T cell activation, the inventor shows here that the IL-18Rα lesion does not affect the activatory functions of Antigen presenting cells (APCs) as TcR Tg T cells proliferated to the same extent when cultured with wt (wild type), IL-18_{-/-} or IL-18Rα_{-/-} Dendritic Cells (DCs).

In contrast to the absence of inflammatory cells in the CNS at the endpoint of EAE the inventor could detect comparable CD4+ T cell infiltration in the IL-18Rα_{-/-} CNS prior to the onset of disease. Other inflammatory cells also infiltrated the CNS to the same extent as in wt and IL-18_{-/-} mice. Therefore the IL-18Rα deficiency does not affect invasion of immune cells into the CNS but must affect their ability to persist. Interestingly, the presence of inflammatory infiltrates in the IL-18Rα_{-/-} CNS, without concomitant EAE susceptibility, resembles the response that occurs in IL-23_{-/-} mice.

The inventor analyzes IL-17 production by IL-18Rα_{-/-} KLH recall lymphocytes and demonstrates that there is indeed a significant decrease in the production of IL-17 at both the RNA and protein levels. Therefore the resistance of IL-18Rα_{-/-} mice to EAE could be explained as a result of insufficient THIL-17 development.

It seemed likely that the lack of THIL-17 cells resulted from the absence of IL-18Ra expression on this subpopulation of T cells. This was not the case, however, as the generation of BM-chimeras demonstrated that only in the presence of RAG_{-/-} BM cells could the susceptibility of IL-18Rα_{-/-} mice (RAG_{-/-} + IL-18Rα^{-/-} > wt) to EAE be rescued. IL-18Rα_{-/-} > wt mice, on the other hand, were resistant to disease induction. Therefore, the presence of IL-18Rα is required on a non-lymphocytic leukocyte and is not directly located on pre-THIL-17 cells. Furthermore, the importance of IL-18Rα on an accessory cell was accentuated in an adoptive transfer experiment whereby encephalitogenic wt T cells could not induce EAE in IL-18Rα_{-/-} mice.

In summary, the inventor shows evidence refuting the TH1 hypothesis of MS and EAE by demonstrating a non-pathogenic role for IL-18 in EAE. In contrast, however, the so-called IL-18Rα is critical for the development of EAE thus implying the presence of an alternative IL-18Rα-binding ligand, which the inventor could confirm by treating IL-18_{-/-} mice with anti-IL-18Rα antibodies thereby diminishing EAE severity. Alternatively, the inventor shows that IL-18Rα signaling is critical for the development of encephalitogenic THIL-17 cells, which thereby explains the resistance of IL-18Rα_{-/-} mice to MOG₃₅₋₅₅-induced EAE.

Basis, in part, for the invention are the results disclosed here above and in the examples of the present application. As explained herein, the inventor of the present invention has discovered that antagonists of IL-18Rα are effective in *vivo* for treating diseases. Specifically, an antibody, which binds the extracellular domain of IL-18Rα, was found to be useful in preventing experimentally-induced MS in a mouse model of this disease. Moreover, the IL-18Rα antagonist also inhibited the progression of an already established disease in the same animal model. Thus, these in *vivo* data indicate that inhibition of IL-18Rα is effective for treating MS. Any method that neutralizes IL-18Ra activity or inhibits expression of the IL-18Rα gene (either transcription or translation) can be used to reduce the MS symptoms.

Accordingly, the invention provides a method of treating, preventing or ameliorating the symptoms of a demyelinating disease (in particular MS), in a subject, in particular a human subject, the method comprising administering to the subject a therapeutically effective amount of an antagonist of IL-18Rα. As used herein, a "therapeutically effective amount" of a compound means the minimum amount of the compound that is effective to treat, ameliorate or prevent the demyelinating disease (in particular MS) or its symptoms. The invention also pertains to the use of an antagonist of IL-18Rα in the manufacture of a medicament for the treatment of a demyelinating disease (in particular MS).

In some embodiments of the present invention, the disease to treat is relapsing-remitting (RR) MS, secondary progressive (SP) MS, primary progressive (PP) MS or progressive relapsing (PR) MS.

The term "antagonist of IL-18Rα" within the context of the present invention refers to an antibody against IL-18Rα wherein the antibody has an antigen binding domain that comprises six CDRs having an amino acid sequence consisting of: SEQ ID NO:5, SEQ ID NO:6, SEQ ID NO:7 SEQ ID NO:8, SEQ ID NO:9 and SEQ ID NO:10, modulating IL-18Rα production and/or action in such a way that IL-18Rα production and/or action is attenuated, reduced, or partially, substantially or completely blocked. In a particular embodiment, the antagonist reduces or prevents the production of IL-18Rα. In another particular embodiment the antagonist partially, substantially or completely blocks the activity of IL-18Rα.

Any antagonists that inhibit IL-18Rα production and/or action in such a way that IL-18Rα production and/or action is attenuated, reduced, or partially, substantially or completely blocked may be used to treat the demyelinating disease (in particular MS) according to the methods of the invention. Accordingly antibodies that selectively bind to IL-18Rα or antibodies that selectively bind to the extracellular domain of IL-18Rα can be 5 used to treat or prevent demyelinating disease (in particular MS).

Accordingly, the invention provides a method of treating, preventing or ameliorating the symptoms of a demyelinating disease (in particular MS), in a subject, in particular a human subject, the method comprising administering to the subject a therapeutically effective amount of an antagonist of IL-18Rα The invention also pertains to the use of an antagonist of IL-18Rα in the manufacture of a medicament for the treatment of a demyelinating disease (in particular MS).

As explained herein, the inventor of the present invention has discovered that antagonists of IL-18Rα are effective in *vivo* for treating diseases. The data obtained by the inventor indicate that inhibition of IL-18Rα is effective for treating MS, in an IL-18 independent manner. Therefore, in an embodiment of the present invention, the antagonists of IL-18Rα used to treat the demyelinating disease (in particular MS) do not inhibit solely IL-18 activity.

The invention also pertains to any of the above or below described antagonist of IL-18Rα for use as a medicament.

In a specific embodiment of the invention, the antagonist is capable of inhibiting the activity of IL18Rα in Antigen presenting cells and more specifically in the Antigen presenting cells selected from the group consisting of monomorphonucleated phagocytes, polymorphonucleated phagocytes, dendritic cells and Natural Killer cells.

In an embodiment of the invention, the antagonist of IL-18Rα is capable of inhibiting the development of IL-17 producing TH cells.

A cDNA encoding human IL-18Rα is presented at SEQ ID NO: 1. This cDNA encodes a 541 amino acids long protein (SEQ ID NO: 2) which includes an extracellular domain of 329 amino acids (residues 1-329 of SEQ ID NO: 2) that includes a signal peptide of 18 amino acids (residues 1-18 of SEQ ID NO: 2), a transmembrane region of 21 amino acids (residues 330 to 350 of SEQ ID NO: 2), and, a cytoplasmic domain from amino acids 351 to 541 of SEQ ID NO: 2.

### 1) Antibodies:

In a preferred embodiment of the present invention, antagonists for use in the methods of the present invention are antibodies that selectively bind to IL-18Rα. Such antibodies are used to treat, prevent or ameliorate the symptoms of a demyelinating disease (in particular MS), in a subject, preferably a human subject. Suitable antibodies include polyclonal antibodies, monoclonal antibodies, human or humanized antibodies, immunoconjugates and antibody fragments (such as Fab, Fab', F(ab')2, and Fv fragments; diabodies; linear antibodies; single-chain antibody molecules; monobodies; and multispecific antibodies formed from antibody fragments) as will be described here after. In a preferred embodiment, the antibodies bind to the extracellular domain of IL-18Rα.

In more specific embodiments, the antibody used in the methods of the present invention selectively binds to the polypeptide of SEQ ID NO: 2, and more specifically to residues 1-329 of SEQ ID NO: 2, residues 19-329 of SEQ ID NO: 2, residues 330 to 350 of SEQ ID NO: 2 or residues 351 to 541 of SEQ ID NO: 2. In a particular aspect, the antibody selectively binds to residues 19-219 of SEQ ID N: 2, or residues 122-329 of SEQ ID N: 2. In another particular aspect, the antibody selectively binds to residues 19-132 of SEQ ID N: 2, or residues 122-219 of SEQ ID N: 2, or residues 213-329 of SEQ ID N: 2. In a preferred embodiment, the antibody selectively binds to an epitope located in the extracellular domain of human IL-18Rα. Even more preferably, this epitope is located into the amino acids 19 to 329 of SEQ ID NO: 2. In another embodiment, this epitope is located into the amino acid residues 19 to 219 of SEQ ID NO: 2, or into amino acid residues 122 to 329 of SEQ ID NO: 2. In another particular aspect, the epitope is located into the amino acids 19-132 of SEQ ID N: 2, or 122-219 of SEQ ID N: 2, or 213-329 of SEQ ID N: 2.

Within the context of this invention, the term "selective" binding indicates that the antibodies preferentially bind the target polypeptide or epitope, i.e., with a higher affinity than any binding to any other antigen or epitope. In other words, binding to the target polypeptide can be discriminated from non-specific binding to other antigens. It is preferred that the antibodies according to the present invention exhibit binding affinity (Ka) to the target polypeptide or epitope of 10⁶ M⁻¹ or greater, preferably 10⁷ M⁻¹ or greater, more preferably 10⁸ M⁻¹ or greater and most preferably 10⁹ M⁻¹ or greater. The binding affinity of an antibody can be readily determined by one of ordinary skill in the art, for example, by Scatchard analysis (Scatchard G., Ann NY Acad. Sci. 51: 660-672, 1949).

Suitable antibodies for use in the methods of the present invention include polyclonal antibodies, monoclonal antibodies, human or humanized antibodies, immunoconjugates and antibody fragments (such as Fab, Fab', F(ab')2, and Fv fragments; diabodies; linear antibodies; single-chain antibody molecules; monobodies; and multispecific antibodies formed from antibody fragments) as will be described here after.

### A. Polyclonal Antibodies:

Methods of preparing polyclonal antibodies from various species, including rodents, primates and horses, have been described for instance in Vaitukaitis et al. (J Clin Endocrinol Metab. 33 (1971) p. 988). Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the polypeptide of SEQ ID NO: 2, or more specifically residues 1-329 of SEQ ID NO: 2, or residues 19-329 of SEQ ID NO: 2, or residues 330 to 350 of SEQ ID NO: 2 or residues 351 to 541 of SEQ ID NO: 2, or a fusion protein thereof, even more specifically residues 19-219 of SEQ ID N: 2, or residues 122-329 of SEQ ID N: 2, or residues 19-132 of SEQ ID N: 2, or residues 122-219 of SEQ ID N: 2, or residues 213-329 of SEQ ID N: 2, or a fusion protein thereof). It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). Repeated injections may be performed. Blood samples are collected and immunoglobulins or serum are separared.

### B. Monoclonal Antibodies

The antibodies may, alternatively, be monoclonal antibodies. The term "Monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. The modifier "monoclonal" indicates the character of the antibody as being obtained from a substantially homogeneous population of antibodies, and is not to be construed as requiring production of the antibody by any particular method.

Methods of producing monoclonal antibodies may be found, for instance, in Harlow et al (Antibodies: A laboratory Manual, CSH Press, 1988) or in Kohler et al (Nature 256 (1975) 495).

In a hybridoma method, a mouse, hamster, or other appropriate host animal, is typically immunized with an immunizing agent (the immunizing agent will typically include the polypeptide of SEQ ID NO: 2, or more specifically residues 1-329 of SEQ ID NO: 2, or residues 19-329 of SEQ ID NO: 2, or residues 330 to 350 of SEQ ID NO: 2 or residues 351 to 541 of SEQ ID NO: 2, or a fusion protein thereof; even more specifically residues 19-219 of SEQ ID N: 2, or residues 122-329 of SEQ ID N: 2, or residues 19-132 of SEQ ID N: 2, or residues 122-219 of SEQ ID N: 2, or residues 213-329 of SEQ ID N: 2, or a fusion protein thereof) to elicit lymphocytes that produce or are capable of producing antibodies that will specifically bind to the immunizing agent. Alternatively, the lymphocytes may be immunized *in vitro*. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, Monoclonal Antibodies: Principles and Practice, Academic Press, (1986) pp. 59-103). Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells. Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, Marcel Dekker, Inc., New York, (1987) pp. 51-63).

The culture medium in which the hybridoma cells are cultured can then be assayed for the presence of monoclonal antibodies directed against the immunizing peptide. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods (Goding, supra). Suitable culture media for this purpose include, for example, Dulbecco's Modified Eagle's Medium and RPMI- 1640 medium. Alternatively, the hybridoma cells may be grown in *vivo* as ascites in a mammal.

The monoclonal antibodies secreted by the subclones may be isolated or purified from the culture medium or ascites fluid by conventional immunoglobulin purificationproceduressuch as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells.

The "monoclonal antibodies" may also be isolated from phage antibody libraries using the techniques described in Clackson et al., Nature, 352:624-628 [1991] and Marks et al., J. Mol. Biol., 222:581-597 (1991), for example.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

Antibodies may also be produced by selection of combinatorial libraries of immunoglobulins, as disclosed for instance in Ward et al (Nature 341 (1989) 544).

### C. Human and Humanized Antibodies:

Preferably, the antibodies for use in the present invention are humanized antibodies or human antibodies. Humanized forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of non-human species (donor antibody) such as mouse, rat or rabbit having the desired specificity, affinity and capacity. In some instances, Fv framework residues of the human immunoglobulin are replaced by corresponding non-human residues.

Methods for humanizing non-human antibodies are well known in the art. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species.

In some cases, transfer of a CDR to a human framework leads to a loss of specificity for the humanized antibody. In these cases, back mutation can be introduced into the framework regions of the human portion of the antibody. Methods of making back mutations are well known in the art and are described in, e.g., Co et al., PNAS USA 88; 2269-2273 (1991) and WO 90/07861.

Human antibodies can also be produced using various techniques known in the art, including phage display libraries (Hoogenboom and Winter, J. Mol. Biol, 227:381 (1991); Marks et al., J. Mol. Biol, 222:581 (1991)). The techniques of Cole et al., and Boerner et al., are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)). Similarly, human antibodies can be made by the introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology, 10: 779-783 (1992); Lonberg et al., Nature, 368: 856-859 (1994); Morrison, Nature, 368: 812-13 (1994); Fishwild et al., Nature Biotechnology, 14:845-51 (1996); Neuberger, Nature Biotechnology, 14: 826 (1996); Lonberg and Huszar, Intern. Rev. Immunol., 13 :65-93 (1995).

### D. Immunoconjugates:

The invention also pertains to the use of immunoconjugates comprising an antibody conjugated to heterologous moieties, such as cytotoxic agents, labels, drugs or other therapeutic agents, covalently bound or not, either directly or through the use of coupling agents or linkers. Cytotoxic agent include chemotherapeutic agent, toxin (e.g., an enzymatically active toxin of bacterial, fungal, plant, or animal origin, or fragments thereof), or a radioactive isotope (i.e., a radioconjugate).

Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re.

In another embodiment, the antibody may be conjugated to a "receptor" (such as streptavidin) for utilization in cells pretargeting wherein the antibody-receptor conjugate is administered to the patient, followed by removal of unbound conjugate from the circulation using a clearing agent and then administration of a "ligand" (e.g., avidin) that is conjugated to a cytotoxic agent (e.g., a radionucleotide).

Moreover, antibodies or antibody fragments of the present invention can be PEGylated using methods in the art and described herein. The antibodies disclosed herein may also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl. Acad. Sci. USA, 77: 4030 (1980); and U.S. Pat. Nos. 4,485,045 and 4,544,545. Liposomes with enhanced circulation time are disclosed in U.S. Patent No. 5,013,556.

### E. Antibody fragments:

The invention also pertains to the use of "Antibody fragments" which comprise a portion of an intact antibody, preferably the antigen binding or variable region of the intact antibody. Examples of antibody fragments include Fab, Fab', F(ab')2, and Fv fragments; diabodies; linear antibodies (Zapata et al., Protein Eng., 8(10): 1057-1062 [1995]); single-chain antibody molecules; monobodies; and multispecific antibodies formed from antibody fragments.

"Fv" is the minimum antibody fragment which contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the VH-VL dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known. The "light chains" of antibodies (immunoglobulins) from any vertebrate species can be assigned to one of two clearly distinct types, called kappa and lambda, based on the amino acid sequences of their constant domains.

"Single-chain antibody molecules" are fragments of an antibody comprising the VH and VL domains of said antibody, wherein these domains are present in a single polypeptide chain. Preferably, the Fv polypeptide further comprises a polypeptide linker between the VH and VL domains which enables the single-chain antibody molecule to form the desired structure for antigen binding. For a review of single-chain antibody molecules, see, Pluckthun in The Pharmacology of Monoclonal Antibodies, Vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (VH) connected to a light-chain variable domain (VL) in the same polypeptide chain (VH - VL). Preferably, by using a linker that is too short to allow pairing between the two domains on the same chain, the domains are forced to pair with the complementary domains of another chain and create two antigen-binding sites. Diabodies are described more fully in, for example, EP 404,097; WO 93/ 11161; and Hollinger et al., Proc. Natl. Acad. Sci. USA, 90:6444-6448 (1993).

The term "monobodies" as used herein, refers to antigen binding molecules with a heavy chain variable domain and no light chain variable domain. A monobody can bind to an antigen in the absence of light chain and typically has three CDR regions designated CDRH1, CDRH2 and CDRH3. Monobodies include "camelid monobodies" obtained from a source animal of the camelid family, including animals with feet with two toes and leathery soles. Animals in the camelid family include camels, llamas, and alpacas. It has been reported that camels (*Camelus dromedaries* and *Camelus bactrianus*) often lack variable light chain domains when material from their serum is analyzed, suggesting that sufficient antibody specificity and affinity can be derived form VH domains (three CDR loops) alone. Monobodies also include modified VH from various animal sources, in particular mammals (for example mouse, rat, rabbit, horse, donkey, bovine or human), which can bind to an antigen in the absence of VL. Preferably, the VH is modified in positions at the VL interface to provide for binding of the VH to antigen in absence of the VL. Davies and Riechmann have for example demonstrated that "camelized monobodies" with high affinity (binding affinity (Ka) to the target polypeptide of 10⁷ M⁻¹ or greater) and high specificity can be generated (Davies & Riechmann, 1995, Biotechnology (N Y), 13(5):475-9). Non-specific binding of the VH through its interface for the light chain variable domain (VL) was prevented through three mutations (G44E, L45R and W47G) in this interface. These mutations were introduced to mimic camelid antibody heavy chains naturally devoid of light chain partners.

### F. Other antibodies for use in the methods of the present invention:

In a preferred embodiment of the present invention, antagonists for use in the methods of the present invention are antibodies as described here above that selectively bind to IL-18Rα. Antibody for use in the methods of the present invention are described for example in the PCT application WO97/31010 or in the European application EP0850952.

Preferably antibodies for use in the methods of the present invention selectively bind to human IL-18Rα and even more preferably to an epitope located in the extracellular domain of human IL-18Rα. Such antibodies are used to treat, prevent or ameliorate the symptoms of a demyelinating diseases (such as MS) in a human subject.

In a more specific embodiment, such antibodies are monoclonal antibodies, human or humanized antibodies, immunoconjugates or antibody fragments (such as Fab, Fab', F(ab')2, and Fv fragments; diabodies; linear antibodies; single-chain antibody molecules; monobodies; and multispecific antibodies formed from antibody fragments) as described here above.

In one particular aspect of the invention, such antibodies selectively bind to the extracellular domain of human IL-18Rα. More specifically, said antibody have an antigen binding domain that comprises six CDRs having the amino acid sequence of SEQ ID NO: 5, SEQ ID NO: 6, SEQ ID NO: 7, SEQ ID NO: 8, SEQ ID NO: 9 and SEQ ID NO: 10.

In another embodiment of the invention, said antibodies comprise a VH domain. Preferably the VH domain comprises or consists in an amino acid sequence of SEQ ID NO: 3. In yet another embodiment the antibody comprises a VL domain. Preferably the VL domain comprises or consists in an amino acid sequence of SEQ ID NO: 4. In a preferred embodiment the antibody comprises a VH and a VL domain. More preferably the antibody comprises a VH domain comprising or consisting in an amino acid sequence of SEQ ID NO: 3 and a VL domain comprising or consisting in an amino acid sequence of SEQ ID NO: 4.

In another embodiment the antibody comprises a heavy chain immunoglobulin constant domain selected from the group consisting of a human IgG1 constant domain; a human IgG2 constant domain; a human IgG3 constant domain; a human IgG4 constant domain; a human IgM constant domain; a human IgE constant domain and a human IgA constant domain. Preferably the heavy chain immunoglobulin constant region domain is a human IgG1 constant domain. In another embodiment the antibody further comprises a light chain immunoglobulin constant domain selected from the group consisting of a human Ig kappa constant domain; and a human Ig lambda constant domain.

In another embodiment the antibody comprises a human IgG1 as Ig constant heavy region; an Ig constant light region selected from the group consisting of a human Ig kappa constant domain; and a human Ig lambda constant domain; an Ig variable heavy region having an amino acid sequence of SEQ ID NO : 3; and an Ig variable light region having an amino acid sequence of SEQ ID NO : 4 (named Ab1 hereafter).

Particularly preferred embodiments of the antibodies disclosed herein are human or humanized antibodies, antibody fragments such as: Fab, Fab', F(ab')2, Fv fragments, diabodies, linear antibodies, single-chain antibodies, monobodies, or multispecific antibodies formed from antibody fragments as defined here above. Most preferably the antibody is a human or humanized antibody.

Other antibodies suitable for use in the method of the present invention include the following monoclonal antibodies: Monoclonal Anti-human IL-18Rα clone 70614 (commercialised by R&D Systems, Minneapolis, MN, USA, catalog number: MAB8401), monoclonal Anti-human IL-18Rα clone 70625 (commercialised by R&D Systems, Minneapolis, MN, USA, catalog number: MAB840), monoclonal anti-human IL-18Rα clone B-E43 (antibody number 80232 (commercialised by Diaclone, Besancon, France, catalog number: 854 900 000)), monoclonal anti-human IL-18Rα clone H44 (antibody number 80438 (commercialised by Diaclone, Besancon, France)). A humanized form of said antibodies is preferred

In one embodiment the antibodies for use in the methods of the invention are antibodies that compete with Ab1, clone 70614, clone 70625, clone B-E43 and/or clone H44 described here above for binding to human IL-18Rα. Competitive binding assays can be used to identify antibodies that compete with Ab1, clone 70614, clone 70625, clone B-E43 and/or clone H44 for binding to human IL-18Rα. Any of a number of competitive binding assays known in the art can be used to measure competition between two antibodies to the same antigen. Briefly, the ability of different antibodies to inhibit the binding of another antibody is tested. For example, antibodies can be differentiated by the epitope to which they bind using a sandwich ELISA assay. This is carried out by using a capture antibody to coat the surface of a well (the capture antibody can be for example: Ab1, clone 70614, clone 70625, clone B-E43 and/or clone H44). A subsaturating concentration of tagged-antigen is then added to the capture surface (e.g. human IL-187Rα or a part of it (e.g. the extracellular domain of human IL-187Ra). This protein will be bound to the antibody through a specific antibody:epitope interaction. After washing a second antibody, which has been linked to a detectable moiety (e.g., HRP, with the labeled antibody being defined as the detection antibody) is added to the ELISA. If this antibody recognizes the same epitope as the capture antibody it will be unable to bind to the target protein as that particular epitope will no longer be available for binding. If however this second antibody recognizes a different epitope on the target protein it will be able to bind and this binding can be detected by quantifying the level of activity (and hence antibody bound) using a relevant substrate. The background is defined by using a single antibody as both capture and detection antibody, whereas the maximal signal can be established by capturing with an antigen specific antibody and detecting with an antibody to the tag on the antigen. By using the background and maximal signals as references, antibodies can be assessed in a pair-wise manner to determine epitope specificity. A first antibody is considered to competitively inhibit binding of a second antibody, if binding of the second antibody to the antigen is reduced by at least 30%, usually at least about 40%, 50%, 55%, 60%, 65%, 70%, 75%, 80% or 85%, and often by at least about 90%, 95%, 96%, 97%, 98% or 99%, in the presence of the first antibody using any of the assays described above. As disclosed hereabove, antibodies that compete with Ab1, clone 70614, clone 70625, clone B-E43 and/or clone H44 for binding to human IL-18Rα, and in particular to the extracellular domain of human IL-187Rα, such as reducing by at least 30%, usually at least about 40%, 50%, 55%, 60%, 65%, 70%, 75%, 80% or 85%, and often by at least about 90%, 95%, 96%, 97%, 98% or 99%, in the presence of Ab1, clone 70614, clone 70625, clone B-E43 and/or clone H44, using any of the assays described above, represent antibodies for use in the methods of the invention. Particularly preferred embodiments of these competitive antibodies are human or humanized antibodies, antibody fragments such as: Fab, Fab', F(ab')2, Fv fragments, diabodies, linear antibodies, single-chain antibodies, monobodies, or multispecific antibodies formed from antibody fragments as defined here above. Most preferably these competitive antibodies are human or humanized antibodies.

In one embodiment of the present invention, the antibodies for use in the methods of the invention are antibodies obtainable or obtained by the following techniques. A host animal (for example a mouse, hamster, rabbit, goat, donkey, monkey or another appropriate host) is immunized with an immunizing agent comprising or consisting of the polypeptide of SEQ ID NO: 2 or a fusion protein thereof, or more specifically comprising or consisting of residues 19-329 of SEQ ID NO: 2 or a fusion protein thereof; even more specifically residues 19-219 of SEQ ID N: 2, or residues 122-329 of SEQ ID N: 2, or residues 19-132 of SEQ ID N: 2, or residues 122-219 of SEQ ID N: 2, or residues 213-329 of SEQ ID N: 2, or a fusion protein thereof, to elicit lymphocytes that produce antibodies that will specifically bind to the immunizing agent. Alternatively, the lymphocytes are immunized *in vitro*. In that case either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent (such as polyethylene glycol), to form a hybridoma cell (Goding, Monoclonal Antibodies: Principles and Practice, Academic Press, (1986) pp. 59-103). The culture medium in which the hybridoma cells are cultured are then be assayed for the presence of monoclonal antibodies directed against the immunizing peptide. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). An ELISA test for the binding of IL-18Rα has been described for example by Vermot-Desroches C, et al Cell Immunol. 2005. 236(1-2):101. After the desired hybridoma cells are identified, the clones may be subcloned by limiting dilution procedures and grown by standard methods (Goding, supra). Suitable culture media for this purpose include, for example, Dulbecco's Modified Eagle's Medium and RPMI- 1640 medium. Alternatively, the hybridoma cells may be grown *in vivo* as ascites in a mammal. The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

Optionally, the antibody obtained may be humanized. Methods for humanizing non-human antibodies are well known in the art. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In some cases, transfer of a CDR to a human framework leads to a loss of specificity for the humanized antibody. In these cases, back mutation can be introduced into the framework regions of the human portion of the antibody. Methods of making back mutations are well known in the art and are described in, e.g., Co et al., PNAS USA 88; 2269-2273 (1991) and WO 90/07861. Alternatively, human antibodies can be made by the introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology, 10: 779-783 (1992); Lonberg et al., Nature, 368: 856-859 (1994); Morrison, Nature, 368: 812-13 (1994); Fishwild et al., Nature Biotechnology, 14:845-51 (1996); Neuberger, Nature Biotechnology, 14: 826 (1996); Lonberg and Huszar, Intern. Rev. Immunol., 13 :65-93 (1995).

In an embodiment, the invention pertains to a method of treating, preventing or ameliorating the symptoms of a demyelinating diseases (such as multiple sclerosis) in a human subject, the method comprising administering to the subject a therapeutically effective amount of an antagonist of IL-18Rα, wherein the antagonist is an antibody that selectively binds to the extra-cellular domain of IL18-Rα and wherein the antibody is obtainable by the process comprising the step or consisting of:
- immunizing a host animal with an immunizing agent comprising residues 19-329 of SEQ ID NO: 2 (or consisting of residues 19-329 of SEQ ID NO: 2, or residues 122-329 of SEQ ID N: 2, or residues 19-132 of SEQ ID N: 2, or residues 122-219 of SEQ ID N: 2, or residues 213-329 of SEQ ID N: 2, or a fusion protein thereof),
- fusing the lymphocytes produced by said host animal with an immortalized cell line to form hybridoma cells,
- selecting clones of hybridoma cells producing antibodies directed against the immunizing peptide,
- testing the activity of the antibodies produced by the different clones of hybridoma cells in an EAE animal model and selecting an antibody that inhibit the progression of EAE in said animal model,
- producing the monoclonal antibody secreted.

Optionally, in a further step, the antibody obtained may be humanized. Alternatively, the host animal immunized is a transgenic animal in which the endogenous immunoglobulin genes have been replaced by human immunoglobulin, e.g., mice in which the endogenous immunoglobulin genes have been partially or completely inactivated, as disclosed here above. Upon challenge, human antibody production is observed, which closely resembles that seen in humans in all respects, including gene rearrangement, assembly, and antibody repertoire.

In another embodiment of the present invention, the antibodies for use in the methods of the invention are antibodies obtainable or obtained by phage display. In that case, the "monoclonal antibodies" are isolated from phage antibody libraries using the techniques described in Clackson et al., Nature, 352:624-628 [1991] and Marks et al., J. Mol. Biol., 222:581-597 (1991), for example.

Preferably, the antibodies described here above and used in the methods of the present invention are human or humanized; techniques for creating such human or humanized antibodies are also well known and are commercially available from, for example, Protein Design Labs, Inc. (Fremont, CA), Medarex Inc, (Princeton, NJ) and Abgennix Inc.(Fremont, CA).

### 2) Pharmaceutical uses of the IL-18Rα antagonists disclosed here above:

The invention pertains to any of the above or below described antagonist of IL-18Rα for use as a medicament. Preferably, any of the above or below described antagonist of IL-18Rα have the ability to reduce the symptoms of a demyelinating disease (such as multiple sclerosis). Therefore, preferably, all the modifications to antagonists of IL-18Rα described herein do not affect significantly their ability to reduce the symptoms of MS. Even more preferably, the modifications to antagonists of IL-18Rα described herein enhance their ability to reduce the symptoms of MS (e.g. by enhancing their half life etc...).

The invention also pertains to methods for treating, preventing or ameliorating the symptoms of a demyelinating disease (such as multiple sclerosis) in a human subject by administering an effective amount of an antagonist of IL-18Rα to the subject. Any antagonists that inhibit IL-18Rα production and/or action in such a way that IL-18Rα production and/or action is attenuated, reduced, or partially, substantially or completely blocked can be used to treat the demyelinating disease (such as multiple sclerosis) according to the methods of the invention. The methods of the present invention include administering an antagonist of IL-18Rα to an individual afflicted with MS, for a period of time sufficient to induce a sustained improvement in the patient's condition. The invention also provides, in part, the use of an antagonist of IL-18Rα in the manufacture of a medicament for the treatment of a demyelinating diseases (such as multiple sclerosis). In some embodiments, the antagonists are the one disclosed here above. For example, antibodies that selectively bind to IL-18Rα, antibodies that selectively bind to the extracellular domain of IL-18Rα can be used to treat or prevent MS. In some embodiments, the disease to treat is relapsing-remitting (RR) MS, secondary progressive (SP) MS, primary progressive (PP) MS or progressive relapsing (PR) MS.

Basis, in part, for the invention is the discovery that inhibitors of IL-18Rα are effective *in vivo* for treating diseases. Specifically, an antibody which binds the extracellular domain of IL-18Rα, was found to be useful in preventing experimentally-induced MS in a mouse model of this disease. Moreover, the IL-18Rα antagonist also inhibited the progression of an already established disease in the same animal model. Thus, these *in vivo* data indicate that inhibition of IL-18Rα is effective for treating MS. Any method that neutralizes IL-18Rα activity or inhibits expression of the IL-18Rα gene (either transcription or translation) can be used to reduce the MS symptoms.

The subject methods involve administering to the patient an IL-18Rα antagonist that is capable of reducing the effective amount of endogenous biologically active IL-18Rα, such as by reducing the amount of IL-18Rα produced, or by preventing its biological activity. Such antagonists include the one disclosed here above.

In a preferred aspect, protein-based therapeutics can be used to inhibit the activity of IL-18Rα protein. For example, preferred methods of the invention utilize antibodies that selectively bind to IL-18Rα or antibodies that selectively bind to the extracellular domain of IL-18Rα as defined here above.

In one preferred embodiment of the invention, sustained-release forms of the antagonist of IL-18Rα described here above, and in particular, antibodies that selectively bind to IL-18Rα, antibodies that selectively bind to the extracellular domain of IL-18Rα described here above, are used. Sustained-release forms suitable for use in the disclosed methods include, but are not limited to, IL-18Rα antagonists that are encapsulated in a slowly-dissolving biocompatible polymer, admixed with such a polymer, and or encased in a biocompatible semi-permeable implant. Degradable polymer microspheres have been designed to maintain high systemic levels of therapeutic proteins. Microspheres are prepared from degradable polymers such as poly(lactide-co-glycolide) (PLG), polyanhydrides, poly (ortho esters), nonbiodegradable ethylvinyl acetate polymers, in which proteins are entrapped in the polymer (Gombotz and Pettit, Bioconjugate Chem. 6:332 (1995); Ranade, "Role of Polymers in Drug Delivery," in Drug Delivery Systems, Ranade and Hollinger (eds.), pages 51-93 (CRC Press 1995); Roskos and Maskiewicz, "Degradable Controlled Release Systems Useful for Protein Delivery," in Protein Delivery: Physical Systems, Sanders and Hendren (eds.), pages 45-92 (Plenum Press 1997); Bartus et al., Science 281:1161 (1998); Putney and Burke, Nature Biotechnology 16:153 (1998); Putney, Curr. Opin. Chem. Biol. 2:548 (1998)). Polyethylene glycol (PEG)-coated nanospheres can also provide carriers for intravenous administration of therapeutic proteins (see, for example, Gref et al., Pharm. Biotechnol. 10:167 (1997)). In addition, the IL-18Rα antagonist can be conjugated with polyethylene glycol (pegylated) to prolong its serum half-life or to enhance protein delivery.

To treat MS, the IL-18Rα antagonist, preferably an antibody that selectively bind to IL-18Rα, or an antibody that selectively bind to the extracellular domain of IL-18Rα, is administered to the patient in an amount and for a time sufficient to induce a sustained improvement in at least one indicator that reflects the severity of the disorder. The degree of improvement is determined based on signs or symptoms, and may also employ questionnaires that are administered to the patient, such as quality-of-life questionnaires. A therapeutically effective amount of an IL-18Rα antagonist is that sufficient to achieve such a sustained improvement.

Improvement might be induced by repeatedly administering a dose of IL-18Rα antagonist until the patient manifests an improvement over baseline for the chosen indicator or indicators. Although the extent of the patient's illness after treatment may appear improved according to one or more indicators, treatment may be continued indefinitely at the same level or at a reduced dose or frequency. Once treatment has been reduced or discontinued, it later may be resumed at the original level if symptoms should reappear.

The pharmaceutical compositions used in the methods of the present invention may contain, in combination with the IL-18Rα antagonist as active ingredient, suitable pharmaceutically acceptable diluents, carriers, biologically compatible vehicles and additives which are suitable for administration to an animal (for example, physiological saline solution) and optionally comprising auxiliaries (like excipients, stabilizers, or adjuvants) which facilitate the processing of the active compounds into preparations which can be used pharmaceutically. The pharmaceutical compositions may be formulated in any acceptable way to meet the needs of the mode of administration. For example, the use of biomaterials and other polymers for drug delivery, as well the different techniques and models to validate a specific mode of administration, are disclosed in literature (Luo B and Prestwich GD, 2001; Cleland JL et al., Curr Opin Biotechnol. (2001), 12(2):212-9). "Pharmaceutically acceptable" is meant to encompass any carrier, which does not interfere with the effectiveness of the biological activity of the active ingredient and that is not toxic to the host to which is administered. For example, for parenteral administration, the above active ingredients may be formulated in unit dosage form for injection in vehicles such as saline, dextrose solution, serum albumin and Ringer's solution. Carriers can be selected also from starch, cellulose, talc, glucose, lactose, sucrose, gelatin, malt, rice, flour, chalk, silica gel, magnesium stearate, sodium stearate, glycerol monostearate, sodium chloride, dried skim milk, glycerol, propylene glycol, water, ethanol, and the various oils, including those of petroleum, animal, vegetable or synthetic origin (peanut oil, soybean oil, mineral oil, sesame oil).

The pharmaceutical composition may be in a liquid or lyophilized form and comprises a diluent (Tris, citrate, acetate or phosphate buffers) having various pH values and ionic strengths, solubilizer such as Tween or Polysorbate, carriers such as human serum albumin or gelatin, preservatives such as thimerosal, parabens, benzylalconium chloride or benzyl alcohol, antioxidants such as ascrobic acid or sodium metabisulfite, and other components such as lysine or glycine. Selection of a particular composition will depend upon a number of factors, including the condition being treated, the route of administration and the pharmacokinetic parameters desired. A more extensive survey of components suitable for pharmaceutical compositions is found in Remington's Pharmaceutical Sciences, 18th ed. A. R. Gennaro, ed. Mack, Easton, PA (1980).

In a preferred embodiment, protein-based therapeutics are used to inhibit the activity of IL-18Rα protein. Preferred methods of the invention utilize antibodies that selectively bind to IL-18Rα, or antibodies that selectively bind to the extra-cellular domain of IL-18Rα as defined here above. Such proteins are administered in the form of a physiologically acceptable composition comprising purified recombinant protein in conjunction with physiologically acceptable carriers, excipients or diluents. Such carriers are non toxic to recipients at the dosages and concentrations employed. Ordinarily, preparing such compositions entails combining the IL-18Rα antagonist with buffers, antioxidants such as ascorbic acid, low molecular weight polypeptides (such as those having fewer than 10 amino acids), proteins, amino acids, carbohydrates such as glucose, sucrose or dextrins, cheating agents such as EDTA, glutathione and other stabilizers and excipients. Neutral buffered saline or saline mixed with conspecific serum albumin are exemplary appropriate diluents. The IL-18Rα antagonist preferably is formulated as a lyophilizate using appropriate excipient solutions (e. g., sucrose) as diluents. Appropriate dosages can be determined in standard dosing trials, and may vary according to the chosen route of administration. In accordance with appropriate industry standards, preservatives may also be added, such as benzyl alcohoL The amount and frequency of administration will depend, of course, on such factors as the severity of the indication being treated, the desired response, the age and condition of the patient, and so forth.

Any accepted mode of administration can be used and determined by those skilled in the art to establish the desired blood levels of the active ingredients. For example, administration may be by various parenteral routes such as subcutaneous, intravenous, intradermal, intramuscular, intraperitoneal, intranasal, transdermal, rectal, oral, or buccal routes. Preferably the pharmaceutical compositions of the invention are administered by injection, either subcutaneous or intravenous. The route of administration eventually chosen will depend upon a number of factors and may be ascertained by one skilled in the art.

The pharmaceutical compositions used in the methods of the present invention can also be administered in sustained or controlled release dosage forms, including depot injections, osmotic pumps, for the prolonged administration of the IL-18Rα antagonist at a predetermined rate, preferably in unit dosage forms suitable for single administration of precise dosages.

Parenteral administration can be by bolus injection or by gradual perfusion over time. Preparations for parenteral administration include sterile aqueous or non-aqueous solutions, suspensions, and emulsions, which may contain auxiliary agents or excipients known in the art, and can be prepared according to routine methods. In addition, suspension of the active compounds as appropriate oily injection suspensions may be administered. Suitable lipophilic solvents or vehicles include fatty oils, for example, sesame oil, or synthetic fatty acid esters, for example, sesame oil, or synthetic fatty acid esters, for example, ethyl oleate or triglycerides. Aqueous injection suspensions that may contain substances increasing the viscosity of the suspension include, for example, sodium carboxymethyl cellulose, sorbitol, and/or dextran. Optionally, the suspension may also contain stabilizers. Pharmaceutical compositions include suitable solutions for administration by injection, and contain from about 0.01 to 99.99 percent, preferably from about 20 to 75 percent of active compound together with the excipient.

It is understood that the dosage administered will be dependent upon the age, sex, health, and weight of the recipient, kind of concurrent treatment, if any, frequency of treatment, and the nature of the effect desired. The dosage will be tailored to the individual subject, as is understood and determinable by one of skill in the art. The total dose required for each treatment may be administered by multiple doses or in a single dose.

In one embodiment of the invention, IL-18Rα antagonist disclosed here above is administered one time per week to treat MS, in another embodiment is administered at least two times per week, and in another embodiment is administered at least once per day. The dose can be from 0.1 to 10 mg/kg, preferably given intravenously as a 15 minutes to 3 hours infusion. The dose is administered repeatedly at biweekly, weekly, or separated by several (2-8 weeks).

If a route of administration of IL-18Rα antagonist other than injection is used, the dose is appropriately adjusted in accord with standard medical practices. For example, if the route of administration is inhalation, dosing may be one to seven times per week at dose ranges from 10 mg/dose to 50 mg per dose.

In many instances, an improvement in a patient's condition will be obtained by injecting a dose of up to about 100 mg of an antibody that selectively bind to IL-18Rα, or an antibody that selectively bind to the extracellular domain of IL-18Rα, as disclosed hereabove, one to three times per week over a period of at least three weeks, though treatment for longer periods may be necessary to induce the desired degree of improvement. The regimen may be continued indefinitely.

### 3) Combination Therapy:

In some embodiments, an antagonist of IL-18Rα, as defined here above, is administered in conjunction with a second therapeutic agent for treating or preventing MS. For example, an antagonist of IL-18Rα (e.g., an antibody that selectively bind to IL-18Rα, or an antibody that selectively bind to the extracellular domain of IL-18Rα, as described here above) may be administered in conjunction with any of the standard treatments for MS including, e.g., corticosteroids, immunosuppressive drugs, neuro-protective agents, immunomodulatory drugs or interferons.

In an embodiment of the present invention, an antagonist of IL-18Rα (e.g., an antibody that selectively bind to IL-18Rα, or an antibody that selectively bind to the extracellular domain of IL-18Rα, as described here above) is administered in conjunction with a corticosteroïd. By "corticosteroid" is meant any naturally occurring or synthetic steroid hormone which can be derived from cholesterol and is characterized by a hydrogenated cyclopentanoperhydrophenanthrene ring system. Naturally occurring corticosteriods are generally produced by the adrenal cortex. Synthetic corticosteriods may be halogenated. Corticosteroids may have glucocorticoid and/or mineralocorticoid activity.

Exemplary corticosteroids include, for example, dexamethasone, betamethasone, triamcinolone, triamcinolone acetonide, triamcinolone diacetate, triamcinolone hexacetonide, beclomethasone, dipropionate, beclomethasone dipropionate monohydrate, flumethasone pivalate, diflorasone diacetate, fluocinolone acetonide, fluorometholone, fluorometholone acetate, clobetasol propionate, desoximethasone, fluoxymesterone, fluprednisolone, hydrocortisone, hydrocortisone acetate, hydrocortisone butyrate, hydrocortisone sodium phosphate, hydrocortisone sodium succinate, hydrocortisone cypionate, hydrocortisone probutate, hydrocortisone valerate, cortisone acetate, paramethasone acetate, methylprednisolone, methylprednisolone acetate, methylprednisolone sodium succinate, prednisolone, prednisolone acetate, prednisolone sodium phosphate, prednisolone tebutate, clocortolone pivalate, flucinolone, dexamethasone 21-acetate, betamethasone 17-valerate, isoflupredone, 9-fluorocortisone, 6-hydroxydexamethasone, dichlorisone, meclorisone, flupredidene, doxibetasol, halopredone, halometasone, clobetasone, diflucortolone, isoflupredone acetate, fluorohydroxyandrostenedione, beclomethasone, flumethasone, diflorasone, fluocinolone, clobetasol, cortisone, paramethasone, clocortolone, prednisolone 21-hemisuccinate free acid, prednisolone metasulphobenzoate, prednisolone terbutate, and triamcinolone acetonide 21-palmitate.

Preferred examples of corticosteroids administered in conjunction with an antagonist of IL-18Rα (e.g., an antibody that selectively bind to IL-18Rα, or an antibody that selectively bind to the extracellular domain of IL-18Rα, as described here above) are prednisone and/or IV methylprednisolone.

In an embodiment of the present invention, an antagonist of IL-18Rα (e.g., an antibody that selectively bind to IL-18Rα, or an antibody that selectively bind to the extracellular domain of IL-18Rα, as described here above) is administered in conjunction with an immunosuppressive drug. In an embodiment of the present invention, the immunosuppressive drug is chosen in the group consisting of methotrexate, azathioprine, cyclophosphamide, and cladribine, which are generally used for severe progressive forms of demyelinating diseases.

In another embodiment of the present invention, an antagonist of IL-18Rα (e.g., an antibody that selectively bind to IL-18Rα, or an antibody that selectively bind to the extracellular domain of IL-18Rα, as described here above) is administered in conjunction with a neuroprotective agent. In an embodiment of the present invention, the neuroprotective agent is chosen in the group consisting of oral myelin, Copaxone (Glatiramer Acetate from Teva), Tysabri (Biogen/Elan), Novantrone (Serono), Teriflunomide (Aventis), Cladribine (Serono/IVAX), 683699 (T-0047) of GSK/Tanabe Seiyaku, Daclizumab (Roche), Laquinimod (Active Biotech) and ZK-117137 (Schering AG). These compounds are all on the market or in clinical trials to treat MS.

In another embodiment of the present invention, an antagonist of IL-18Rα (e.g., an antibody that selectively bind to IL-18Rα, or an antibody that selectively bind to the extracellular domain of IL-18Rα, as described here above) is administered in conjunction with an immunomodulatory drug. In this respect, a particular immunomodulatory drug for use in the present invention include FTY720 (2-amino-2-[2-(4-octylphenyl)ethyl]-1,3-propanediol, fingolimod). FTY720 which is in phase II to treat MS (Novartis) has the following formula:

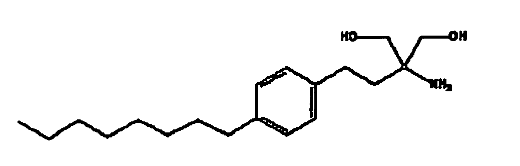

FTY720 has been identified as an orally active immunosuppressant (see, e.g., WO 94/08943; WO 99/36065) obtained by chemical modification of myriocin. Other immunomodulatory drugs for use in the present invention, include derivatives of FTY720. Derivatives of FTY720 include 2-amino-1,3-propanediol compounds as described in WO94/08943, having the following formula, as well as any pharmaceutically acceptable salts thereof: 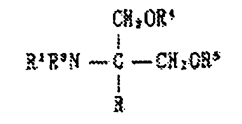 wherein R is an optionally substituted straight- or branched carbon chain which may have, in the chain, a bond, a hetero atom or a group selected from the group consisting of a double bond, a triple bond, oxygen, sulfur, sulfinyl, sulfonyl, -N(R6)-where R6 is hydrogen, alkyl, aralkyl, acyl or alkoxycarbonyl, carbonyl, optionally substituted arylene, optionally substituted cycloalkylene, optionally substituted heteroarylene and an alicycle thereof, and which may be substituted, at the chain end thereof, by a double bond, a triple bond, optionally substituted aryl, optionally substituted cycloalkyl, optionally substituted heteroaryl or an alicycle thereof; an optionally substituted aryl, an optionally substituted cycloalkyl, an optionally substituted heteroaryl or an alicycle thereof; and

R2, R3, R4 and R5 are the same or different and each represents a hydrogen, an alkyl, an aralkyl, an acyl or an alkoxycarbonyl or, R4 and R5 may be bonded to form an alkylene chain which may be substituted by an alkyl, aryl or aralkyl.

The above, optionally substituted straight- or branched carbon chains, may have a substituent selected from the group consisting of alkoxy, alkenyloxy, alkynyloxy, aralkyloxy, alkylenedioxy, acyl, alkylamino, alkylthio, acylamino, alkoxycarbonyl, alkoxycarbonylamino, acyloxy, alkylcarbamoyl, haloalkyl, haloalkoxy, nitro, halogen, amino, hydroxyimino, hydroxy, carboxy, optionally substituted aryl, optionally substituted aryloxy, optionally substituted cycloalkyl, optionally substituted heteroaryl and an alicycle thereof; the aforementioned optionally substituted arylene, optionally substituted cycloalkylene, optionally substituted heteroarylene and an alicycle thereof may have a substituent selected from the group consisting of alkoxy, alkenyloxy, alkynyloxy, aralkyloxy, alkylenedioxy, acyl, alkylamino, alkylthio, acylamino, alkoxycarbonyl, alkoxycarbonylamino, acyloxy, alkylcarbamoyl, haloalkyl, haloalkoxy, nitro, halogen, amino, hydroxy and carboxy; and the optionally substituted aryl, optionally substituted aryloxy, optionally substituted cycloalkyl, optionally substituted heteroaryl and an alicycle thereof may have a substituent selected from the group consisting of alkyl, alkoxy, alkenyloxy, alkynyloxy, aralkyloxy, alkylenedioxy, acyl, alkylamino, alkylthio, acylamino, alkoxycarbonyl, alkoxycarbonylamino, acyloxy, alkylcarbamoyl, haloalkyl, haloalkoxy, nitro, halogen, amino, hydroxy and carboxy.

Specific examples of such 2-amino-1,3-propanediol compounds include 2-amino-2-[2-(4-heptylphenyl)ethyl]-1,3-propanediol, 2-amino-2-[2-(4-octylphenyl)ethyl]-1,3-propanediol, 2-amino-2-[2-(4-nonylphenyl)ethyl]-1,3-propanediol 2-amino-2-[2-(4-decylphenyl)ethyl]-1,3-propanediol, 2-amino-2-[2-(4-undecylphenyl)ethyl]-1,3-propanediol, 2-amino-2-[2-(4-dodecylphenyl)ethyl]-1,3-propanediol, 2-amino-2-[2-(4-tridecylphenyl)-ethyl]-1,3-propanediol, 2-amino-2-[2-(4-tetradecylphenyl)ethyl]-1,3-propanediol, 2-amino-2-[2-(4-hexyloxyphenyl)ethyl]-1,3-propanediol, 2-amino-2-[2-(4-heptyloxyphenyl)ethyl]-1,3-propanediol, 2-amino-2-[2-(4-octyloxyphenyl)ethyl]-1,3-propanediol, 2-amino-2-[2-(4-nonyloxyphenyl)ethyl]-1,3-propanediol, 2-amino-2-[2-(4-decyloxyphenyl)ethyl]-1,3-propanediol, 2-amino-2-[2-(4-undecyloxyphenyl)ethyl]-1,3-propanediol, 2-amino-2-[2-(4-dodecyloxyphenyl)ethyl]-1,3-propanediol, 2-amino-2-[2-(4-tridecyloxyphenyl)ethyl]-1,3-propanediol, 2-amino-2-[2-(4-(8-fluorooctyl)phenyl)ethyl]-1,3-propanediol, 2-amino-2-[2-(4-(12-fluorododecyl)phenyl)ethyl]-1,3-propanediol, 2-amino-2-[2-(4-(7-fluoroheptyloxy)phenyl)ethyl]-1,3-propanediol, 2-amino-2-[2-(4-(11-fluoroundecyloxy)phenyl)ethyl]-1,3-propanediol, 2-amino-2-[2-(4-(7-octenyloxy)phenyl)ethyl]-1,3-propanediol, 2-amino-2-[2-(4-heptylphenyl)ethyl]-1,3-propanediol, 2-amino-2-[2-(4-octylphenyl)ethyl]-1,3-propanediol, 2-amino-2-[2-(4-nonylphenyl)ethyl]-1,3-propanediol, 2-amino-2-[2-(4-decylphenyl)ethyl]-1,3-propanediol, 2-amino-2-[2-(4-undecylphenyl)ethyl]-1,3-propanediol, 2-amino-2-[2-(4-dodecylphenyl)ethyl]-1,3-propanediol, 2-amino-2-[2-(4-heptyloxyphenyl)ethyl]-1,3-propanediol, 2-amino-2-[2-(4-octyloxyphenyl)ethyl]-1,3-propanediol, 2-amino-2-[2-(4-nonyloxyphenyl)ethyl]-1,3-propanediol, 2-amino-2-[2-(4-undecyloxyphenyl)ethyl]-1,3-propanediol, or 2-amino-2-[2-(4-(7-octenyloxy)phenyl)ethyl]-1,3-propanediol, as well as any pharmaceutically acceptable salts thereof.

In another embodiment of the present invention, an antagonist of IL-18Rα (e.g., an antibody that selectively bind to IL-18Rα, or an antibody that selectively bind to the extracellular domain of IL-18Rα, as described here above) is administered in conjunction with an interferon. In this respect, a particular interferon for use in the present invention is interferon-beta. The terms "interferon (IFN)" and "interferon-beta (IFN-beta)", as used herein, are intended to include fibroblast interferon in particular of human origin, as obtained by isolation from biological fluids or as obtained by DNA recombinant techniques from prokaryotic or eukaryotic host cells, as well as their salts, functional derivatives, variants, analogs and active fragments. A particular type of interferon beta is interferon beta-1a.

The use of interferons of human origin is preferred in accordance with the present invention. IFN-beta suitable in accordance with the present invention is commercially available, e.g., as Rebif® (Serono), Avonex® (Biogen) or Bertaseron/Betaferon® (Schering). Rebif® (recombinant human interferon-) is the latest development in interferon therapy for multiple sclerosis (MS) and represents a significant advance in treatment. Rebif® is interferon (IFN)-beta 1a, produced from mammalian cell lines. It was established that interferon beta-1a given subcutaneously three times per week is efficacious in the treatment of Relapsing-Remitting Multiple Sclerosis (RRMS). Interferon beta-1a can have a positive effect on the long-term course of MS by reducing number and severity of relapses and reducing the burden of the disease and disease activity as measured by MRI. Particular examples of interferon administered in conjunction with IL-18Rα antagonist for use in the methods of the present invention therefore are Rebiff® (Serono), Avonex® (Biogen) or Bertaseron/Betaferon® (Schering).

A particular aspect of the invention pertains to a method of treating MS, particularly relapsing-remitting (RR) MS, secondary progressive (SP) MS, primary progressive (PP) MS or progressive relapsing (PR) MS, in a subject in need of such treatment, comprising administering to the subject a therapeutically effective amount of a combination of an antagonist of IL-18Rα as disclosed here above and a corticosteroid, an immunosuppressive drug, a neuro-protective agent, an immunomodulatory drug or an interferon as disclosed here above. In certain embodiments the cortisteroid is prednisone or IV methylprednisolone. In certain embodiments the immunosuppressive drug is methotrexate, azathioprine, cyclophosphamide or cladribine. In certain embodiments the neuroprotective agent is oral myelin, Copaxone, Tysabri, Novantrone, Teriflunomide, Cladribine, 683699 (T-0047), Daclizumab, Laquinimod or ZK-117137. In certain embodiments the immunomodulatory drug is 2-amino-2-[2-(4-octylphenyl)ethyl]-1,3-propanediol (FTY720). In certain embodiments the interferon is interferon beta-1a (in particular Rebif® (Serono)).

The antagonist of IL-18Rα (e.g., an antibody that selectively bind to IL-18Rα, or an antibody that selectively bind to the extracellular domain of IL-18Rα, as described here above) and the second therapeutic agent as disclosed here above may be administered simultaneously, separately or sequentially. For example, the antagonist of IL-18Rα may be administered first, followed by the second therapeutic agent. Alternatively, the second therapeutic agent may be administered first, followed by the antagonist of IL-18Rα. In some cases, the antagonist of IL-18Rα and the second therapeutic agent are administered in the same formulation. In other cases the antagonist of IL-18Rα and the second therapeutic agent are administered in different formulations. When the antagonist of IL-18Rα and the second therapeutic agent are administered in different formulations, their administration may be simultaneous or sequential.

The invention further pertains to product comprising any of the above or below described antagonist of IL-18Rα, and a corticosteroïd, immunosuppressive drug, neuro-protective agent, immunomodulatory drug or interferon, as disclosed here above, as a combined preparation for simultaneous, separate or sequential use in the therapy of MS in a mammalian subject, preferably a human subject.

Further aspects and advantages of the present invention will be disclosed in the following examples.

### EXAMPLES

### Example 1: p35 _{-/-} IL-18_{-/-} double knockout mice are susceptible to EAE

It has previously been shown that deletion of IL-12p35, renders mice hypersusceptible to MOG(myelin oligodendrocyte glycoprotein)-peptide-induced Experimental Autoimmune Encephalomyelitis (EAE) in mice (Becher,B., et al. J. Clin. Invest 110, 493-497 (2002)). IL-18 acts in synergy with IL-12 to polarize Th1 cells (type 1 helper T cells) and Shi *et al.* have produced evidence demonstrating that mice deficient in IL-18 are resistant to EAE (Shi,F.D.,et al., J. Immunol. 165, 3099-3104 (2000)).

To assess whether IL-18 is capable of compensating for the loss of IL-12 in p35_{-/-} mice, thus leading to their EAE susceptibility, we generated mice deficient in both IL-12p35 and IL-18 (p35_{-/-} X IL-18_{-/-}).

Mice (n = 5 mice/group) were immunized subcutaneously with 200 µg of MOG₃₅₋₅₅ peptide (amino acid sequence: MEVGWYRSPFSRVVHLYRNGK (SEQ ID NO: 11)), obtained from GenScript, emulsified in CFA (DIFCO, Detroit, MI). Mice received 200 ng pertussis toxin (Sigma-Aldrich) intraperitoneally at the time of immunization and 48 hours later.

Mice were scored daily as follows: 0) no detectable signs of EAE; 0.5) distal tail limp; 1) complete tail limp; 2) unilateral partial hind limb paralysis; 2.5) bilateral partial limb paralysis; 3) complete bilateral hind limb paralysis; 3.5) complete hind limb paralysis and unilateral forelimb paralysis; 4) total paralysis of fore and hind limbs (score > 4 to be euthanized); 5) death. Each time point shown is the average disease score of each group. Statistical significance was assessed using an unpaired Student's t-Test. Immunization with MOG₃₅₋₅₅ emulsified in CFA showed that p35_{-/-} x IL-18_{-/-} mice are fully susceptible to EAE and have a similar disease score and development as is produced in wt (see Figure 1a). Therefore, the lack of protection generated by IL-18 deletion in p35_{-/-} mice shows that IL-18 is not responsible for inducing EAE susceptibility in p35_{-/-} mice but it also implies that IL-18 itself is a cytokine that has little or no effect in EAE pathogenesis.

### Example 2: TL-18_{-/-}, but not IL-18Rα_{-/-}, mice are susceptible to EAE

As our experiments in p35_{-/-} x IL-18_{-/-} mice seemed to contradict the previously proposed pathogenic role for IL-18 in EAE, we actively immunized wt and IL-18_{-/-} mice with MOG peptide (as described in example 1) and found that IL-18_{-/-} mice were fully susceptible to EAE and indeed had a clinical score and disease progression comparable to that of the wt mice (see Figure 1b and Table 1).

Mice deficient in IL-18Rα have been described as having a phenotype similar to that of IL-18_{-/-} mice in that IFNγ production is reduced. Interestingly, and in sharp contrast to both wt and IL-18_{-/-} mice, IL-18Rα_{-/-} mice were completely resistant to EAE induction (see Figure 1b and Table 1).

Histological analysis of the spinal cords from wt, IL-18_{-/-} and IL-18Rα_{-/-} mice obtained after EAE induction demonstrated that leukocyte infiltration into the CNS correlated well with clinical severity of disease.

To do so, mice were euthanized with CO2, followed by perfusion with PBS and subsequent perfusion with 4% paraformaldehyde (PFA) in PBS. The spinal column was removed and fixed in 4% PFA in PBS. The spinal cord was then dissected and paraffin-embedded prior to staining with either haematoxylin & eosin or CD3, B220 and MAC-3 antibodies (BD Pharmingen) to assess infiltration of inflammatory cells or luxol fast blue to determine the degree of demyelination.

EAE-susceptible wt and IL-18_{-/-} mice had significant inflammation, characterized by infiltration of inflammatory cells (Figure 2a) such as T cells (Figure 2c), macrophages (Figure 2e) and B cells (Figure 2d), and demyelination (Figure 2b), while there was no presence of inflammatory infiltrates or demyelination in the spinal cord of EAE-resistant IL-18Rα_{-/-} mice (Figure 2a-e).

To verify the inability of IL-18_{-/-} mice to secrete IL-18, we extensively verified the targeting strategy and genotype of the mice and could clearly establish that IL-18_{-/-} mice do not contain IL-18 mRNA or protein. We also analyzed whether we could detect IL-18 secreted from activated splenocytes derived from wt and IL-18_{-/-} mice, which showed that IL-18_{-/-} mice are indeed completely IL-18 deficient in contrast to wt mice (See Figure 3).

As it has been observed in many experimental systems that deletion of IL-18 consistently results in the paucity of an IFNγ response (Wei,X.Q. et al. J. Immunol. 163, 2821-2828 (1999), Kinjo,Y. et al. J. Immunol. 169, 323-329 (2002)), we stimulated lymphocytes derived from naive wt, IL-18_{-/-} and IL-18Rα-/- mice *in vitro* with the lectin Concanavalin A (ConA) for 16 hours and IFN-γ production was subsequently measured by ELISA.

To do so, axillary and inguinal lymph nodes (LN) were isolated from naive mice. 2x10⁵ cells were placed as triplicates in a 96-well plate. 5 µg/ml ConA was used for stimulation for 16 hours and IFN-γ production was subsequently measured by ELISA (Pharmingen, La Jolla, CA).

Consistent with the principle that IL-18 has an effect on IFNγ production, LN cells from both IL-18_{-/-} and IL-18Rα_{-/-} mice did not secrete IFNγ in contrast to the wt LN cells (Figure 4a).

### Example 3: Blocking IL-18Rα prevents EAE in IL-18_{-/-} mice

The discordant behavior of IL-18- and IL-18Rα-deficient mice with regards to EAE strongly points towards an additional IL-18Rα ligand with powerful encephalitogenic properties. In order to assess whether IL-18Rα and IL-18 have independent biological functions, we blocked IL-18Rα in EAE-susceptible IL-18_{-/-} mice.

Mice (n = 5 mice/group) were immunized subcutaneously with 200 µg of MOG₃₅₋₅₅ peptide (amino acid sequence: MEVGWYRSPFSRVVHLYRNGK (SEQ ID NO: 11)), obtained from GenScript, emulsified in CFA (DIFCO, Detroit, MI) Mice received 200 ng pertussis toxin (Sigma-Aldrich) intraperitoneally at the time of immunization and 48 hours later. Monoclonal anti-IL-18Rα antibody (clone 112624) (R&D Systems) was or was not administered either 1 day pre-immunization (450 µg/mouse) and every 3 days thereafter (300 µg/mouse) or every 3 days beginning from disease onset (300 µg/mouse).

Mice were scored daily as follows: 0) no detectable signs of EAE; 0.5) distal tail limp; 1) complete tail limp; 2) unilateral partial hind limb paralysis; 2.5) bilateral partial limb paralysis; 3) complete bilateral hind limb paralysis; 3.5) complete hind limb paralysis and unilateral forelimb paralysis; 4) total paralysis of fore and hind limbs (score > 4 to be euthanized); 5) death.

Each time point shown is the average disease score of each group. Statistical significance was assessed using an unpaired Student's t-Test.

Treatment of IL-18_{-/-} mice with anti-IL-18Rα mAbs, given 1 day pre-immunization and every 3 days thereafter until the end of the experiment, significantly reduced disease development (Figure 5a). Administration of anti-IL-18Rα mAbs did not lead to deletion of IL-18Rα-expressing cells nor did it alter the composition of peripheral leukocytes in the blood, LN or spleen (see Figure 11).

Combining the facts that IL-18Rα antagonists prevent EAE even in mice in which its ligand is completely removed by gene-targeting and that IL-18 has reportedly only a low affinity to IL-18Rα, we propose that another ligand must be responsible for the engagement, signaling and immune development mediated by IL-18Rα.

Interestingly, treating IL-18_{-/-} mice with antagonistic mAbs post-immunization (day 10 p.i.) also abrogated EAE progression (Figure 5b) and this occurred to the same extent as Abs administered prior to immunization suggesting that IL-18Rα engagement is an important event during the effector phase of EAE.

### Example 4: Mitogen-, but not Ag-driven activation requires IL-18 for Th1 polarization

Given the dichotomy between IL-18_{-/-} and IL-18Rα^{-/-} mice with regards to EAE susceptibility, we wanted to determine the ability of both mice to properly prime and polarize naïve T cells towards an effector phenotype. Wt, IL-18^{-/-} and IL-18Rα^{-/-} mice were immunized subcutaneously with KLH and 7 days later lymphocytes were isolated and subsequently restimulated with KLH *in vitro.*

To do so axillary and inguinal lymph nodes were isolated from mice primed by injections of 100 µg/flank of Keyhole limpit hemocyanin (KLH) (Sigma) emulsified in CFA 7 days earlier. 2x10⁵ cells were placed as triplicates in a 96-well plate. KLH recall cells were stimulated for 48 hours with 50 µg/ml KLH, 5 µg/ml ConA or medium and 0.5µCi/ml 3[H]-thymidine was added after 24 hours to observe proliferative responses. Thymidine incorporation was assessed using a Filtermate Harvester and a scintillation and luminescence counter. For cytokine analysis, the culture supernatant of sister cultures was harvested after 48 hours and analyzed for IFNγ production by ELISA (Pharmingen, La Jolla, CA) and overall cytokine/chemokine secretion by cytokine array (Raybiotech).

Surprisingly, we did not observe any significant difference in the IFNγ-producing ability of IL-18_{-/-} and IL-18Rα_{-/-} mice and the levels of IFNγ produced by lymphocytes derived from IL-18_{-/-} or IL-18Rα_{-/-} mice were identical to that of cells obtained from wt mice (Figure 4b). Furthermore, the proliferative capacity of Ag-driven lymphocytes between the different mouse strains was identical (Figure 4c). Our data support the notion that IL-18 is a critical co-factor for the early IFNγ response of freshly polyclonally activated T cells (Figure 4a) yet Ag-driven Th1 polarization is more dependent on IL-12 alone and thus IL-18 independent.

Although T_{H}1 development appeared unaffected in IL-18Rα_{-/-} mice we next wanted to assess the capacity of IL-18Rα-deficient Antigen-Presenting cells (APC's) to prime naive T cells.

To do so, we co-cultured mature, SMARTA peptide (p11)-pulsed wt, IL-18_{-/-} and IL-18Rα_{-/-} BM (Bone Marrow)-derived Dendritic Cells (DC's) with SMARTA-TcR-transgenic CD4+ T cells and measured proliferation by thymidine incorporation (Figure 4d).

The protocol used was the following:
**Generation of BM-derived DC's:** BM-donor mice were euthanized using CO₂ and femur and tibia were removed. BM-cells were isolated by flushing the bones with PBS and were filtered through a 100µm cell strainer. Cells (2-2.5x10⁶ in 10 ml) were cultured in complete RPMI with the addition of 10% GM-CSF. After at least 6 days, BM-derived DC's were matured with 10µg/ml lipopolysaccharide (LPS) overnight while immature BM-derived DC's are maintained in GM-CSF-containing medium. On at least day 7, BM-derived DC's were used experimentally.
**Tansgenic (Tg) T cell proliferation:** For *in vitro* proliferation of transgenic T cells, spleens are isolated from naïve TcR Tg mice and CD4+ T cells are purified using BD Biomag magnetic beads. The purity of T cell isolation is verified by FACS analysis. 1x10⁵ Smarta T cells were cultured in a 96-well plate together with 300-30,000 immature or mature bone-marrow derived dendritic cells. Prior to co-culture, BM-derived DCs were pulsed with 1 µg/ml SMARTA p11 peptide (GPDIYKGVYQFKSVEFD (SEQ ID NO: 12)) (GenScript) in RPMI for 3 hours, followed by washing and irradiation with 2000 rads. Non-pulsed DCs were used as a control as well as T cells cultured alone. Cells were incubated for 4 days and ³[H]-thymidine was added for the last 18 hours of culture.

No significant difference in T cell priming was observed even when immature DC's were used to activate SMARTA T cells.

Even though the above data imply that there is no deficiency in the ability of IL-18Rα_{-/-} DC's and T cells to become activated, we decided to confirm the activation status of both cell types at the level of activation marker expression. We looked at expression markers on LPS matured DC's as well as KLH-restimulated T cells by FACS, which showed that there is no difference in upregulation of CD80, CD86 and CD40 on IL-18Rα_{-/-} DC's and also no difference in CD5, CD62L and CD44 expression by IL-18Rα_{-/-} T cells, in comparison to wt and IL-18_{-/-} cells (Figure 6). Therefore the IL-18Rα lesion does not affect T cell or DC activation, at least not at the level of upregulation of surface markers required for adequate stimulation.

### Example 5: IL-18Rα_{-/-} CD4₊ T cells invade the CNS during EAE

EAE is characterized by a massive influx of inflammatory cells into the CNS at the peak of disease yet immune cells also invade the CNS prior to the onset of clinical symptoms (Hickey,W.F. Brain Pathol. 1, 97-105 (1991), Wekerle,H., et al., J. Exp. Biol. 132, 43-57 (1987)). For example, recruitment of CD4+ T cells into the CNS is critical for the initiation of the effector phase of EAE yet the infiltration of polymorphonuclear leukocytes into the CNS appears to have a role in orchestrating these events (McColl,S.R. et al., J. Immunol. 161, 6421-6426 (1998)). Therefore in order to establish whether IL-18Rα_{-/-} inflammatory cells are completely absent from the CNS at timepoints of pre-clinical disease, we immunized mice and analyzed the CNS for inflammatory infiltrates on days 5, 7 and 9 post-immunization.

In contrast to the lack of immune cells at the end-point of disease in IL- 18Rα_{-/-} mice (Figure 2a-e), IL-18Rα_{-/-} CD4₊ T cells were capable of CNS infiltration to the same extent as those of wt and IL-18_{-/-} mice on days 5, 7 and 9 post-immunization, as analyzed by flow cytometry (Figure 7). There were also comparable numbers of granulocytes, macrophages and B cells present in the CNS. However, as seen in figure 2, there is a significant difference in the presence of IL-18Rα_{-/-} inflammatory cells in the CNS at timepoints of clinical disease thus demonstrating their inability to persist during the effector phase of EAE. Interestingly, these results reflect data obtained in IL-23p19_{-/-} mice, which are also resistant to MOG₃₅₋₅₅-induced EAE and in which the deficiency does not prevent infiltration of inflammatory cells into the CNS, as observed on day 7 post-immunization (Langrish,C.L. et al., J. Exp. Med. 201, 233-240 (2005)).

### Example 6: Lack of IL-18Rα prevents IL-17 production

The similarities between IL-18Rα_{-/-} and IL-23_{-/-} mice with regards their EAE resistance with concomitant inflammatory cell invasion into the CNS, provoked us to assess the impact of IL-18Rα on IL-17 production in our mice. IL-17 producing T_{H} cells (T_{H}IL-17) are now accepted to be the main pathogenic population during autoimmune inflammation. To define differences between EAE-susceptible IL-18_{-/-} and EAE-resistant IL-18Rα_{-/-} mice with regards to cytokine secretion, we used a cytokine-protein array (Raybiotech) allowing the simultaneous analysis of 62 different cytokines secreted by lymphocytes upon encountering their cognate recall Ag.
wt, IL-18_{-/-} and IL-18Rα_{-/-} mice were immunized with KLH and 7 days later, lymphocytes were isolated and restimulated with 50 µg/ml KLH (see figure 8).

In comparison to IL-18_{-/-} lymphocytes, IL-18Rα_{-/-} lymphocytes produced much less IL-17. To confirm this finding, we analyzed the levels of this cytokine at both the RNA and protein level. Real-time PCR of RNA taken from lymphocytes upon restimulation with KLH showed that the expression of both IL-17 mRNA is significantly decreased in the IL-18Rα_{-/-} cells in comparison to wt and IL-18_{-/-} cells (Figure 8a). These findings were corroborated by IL-17 ELISA using the supernatant of the same KLH restimulated cells (Figure 8b).

### Example 7: The IL-18Rα lesion affects cells in the accessory cell immune compartment

The lack of IL-18Rα completely prevents the development of EAE via the prevention of T_{H}IL-17 development, whereas its putative ligand IL-18 appears to be irrelevant.

The cell type on which the IL-18Rα exerts its primary effects remains unknown. This is mainly due to the fact that IL-18Rs are expressed by various cell types and tissues. However, one is likely to presume that the presence of IL-18Rα on CD4+ T cells is absolutely critical for the subsequent polarization of T_{H}IL-17 cells. In order to identify the cell and tissue location of the IL-18Rα lesion in EAE, we selectively expressed IL-18Rα on cells in the leukocyte compartment using irradiation bone-marrow (BM)-chimeras.

### Irradiation Bone Marrow (BM)-chimeric mice:

BM-donor mice were euthanized using CO2 and BM-cells were isolated by flushing femur, tibia, radius and hip bones with phosphate buffered solution (PBS). BM cells are then passed through a 100 µm cell strainer and cells are washed with PBS. Recipient mice are lethally irradiated with 1100 rads (split dose) and i.v. injected with 12-25x10⁶ BM-cells. Engraftment takes place over 8 weeks of recovery.

Following irradiation and reconstitution, the APC compartment in secondary lymphoid tissues of recipient mice is comprised entirely of BM cells derived from donor mice (Becher,B., et al., J. Exp. Med. 193, 967-974 (200 1)).
We generated BM chimeras by transferring either a 4:1 ratio of RAG_{-/-} and IL-18Rα_{-/-} BM into wt recipients (RAG_{-/-} + IL-18Rα_{-/-} → wt) or IL-18Rα_{-/-} BM only into wt recipients (IL-18Rα-/- → wt). wt-BM was transferred into wt recipients as a control (wt → wt) (Table 2).

RAG_{-/-} mice do not have lymphocytes and the resulting chimera (RAG_{-/-} + IL-18Rα_{-/-} → wt) thus has an IL-18Rα-deficient lymphocyte compartment, whereas the majority of all other leukocytes has undisrupted IL-18Rα alleles.

As expected IL-18Rα_{-/-}→wt mice were resistant to EAE upon immunization with MOG peptide. Alternatively, addition of BM from RAG_{-/-} mice, which has no T or B cells and therefore expresses IL-18Rα only on accessory cells, not on lymphocytes, was able to overcome the resistance of IL-18Rα_{-/-} mice to EAE (Figure 9). Thus IL-18Rα must exert its primary effects in the accessory cell (mono- and polymorphonucleated phagocytes, DC's & NK-cells) compartment. Again, this finding is highly unexpected, given that IL-18 is thought to exert its effects on T cells and NK cells, but it is completely consistent with our observations so far.

### Example 8: Lack of IL-18Rα on host cells prevents EAE development induced by the adoptive transfer of MOG-reactive T cells

The above data indicate that the lack of IL-18Rα on accessory cells does not influence T_{H} cell priming and expansion. Furthermore, RAG_{-/-} + IL-18Rα_{-/-}→wt mixed BM-chimeras (Figure 9) clearly demonstrate that the IL-18Rα deficiency lesions accessory cell function vital for the development of EAE. We subsequently performed an adoptive transfer experiment to reveal the role and function of IL-18R signaling in accessory cells during EAE. To do so, we adoptively transferred encephalitogenic MOG-reactive T cells derived from wt donor mice into groups of both wt and IL-18Rα_{-/-} recipient mice. As expected, fully primed and activated encephalitogenic T cells derived from wt mice induced EAE in wt recipient mice, yet they were incapable of inducing clinical EAE in IL-18Rα-deficient hosts (Figure 10). This finding again underlines that the IL-18Rα-deficiency lesions a non-lymphocytic leukocyte of the host which is essential for the development of EAE, independent of T cell activity.

**Table 1**

| IL-18R is critical for the development of active EAE in mice | | | |
|---|---|---|---|
| **Mouse genotypes** | **incidence (%)** | **Moan day of disease onset** | **Mean maximal clinical score (+/- SEM)** |
| Wt | 17/20 (85) | 11.8 | 2.6 +/- 0.13 |
| IL-18-/- | 20/22(91) | 12.8 | 2.35 +/- 0.13 |
| IL-18R-/- | 2/20 (10) | 18.5 | 2.6 +/- 0.12 |

| | | | |
|---|---|---|---|
| * of diseased animals | | | |

**Table 2**

| **Donor bone-marrow** | **Recipient Mouse** | **IL-18R Deficiency** |
|---|---|---|
| Wt | Wt | No lesion |
| IL-18R-/- | Wt | All cells |
| RAG-/- + IL-18R-/- (1:4) | Wt | Lymphocytes |

## Claims

1. Use of an antibody against IL-18Rα wherein the antibody has an antigen binding domain that comprises six CDR(s) having an amino acid sequence consisting of: SEQ ID NO: 5 representing CDR1 Heavy chain, SEQ ID NO: 6 representing CDR2 Heavy chain, SEQ ID NO: 7 representing CDR3 Heavy chain, SEQ ID NO: 8 representing CDR1 light chain, SEQ ID NO: 9 representing CDR2 light chain and SEQ ID NO: 10 representing CDR3 light chain in the manufacture of a medicament for the treatment of a demyelinating disease.

2. Use according to claim 1 wherein said demyelinating disease is multiple sclerosis.

3. The use according to claims 1 or 2 wherein the subject is affected by relapsing-remitting (RR) multiple sclerosis, secondary progressive (SP) multiple sclerosis, primary progressive (PP) multiple sclerosis or progressive relapsing (PR) multiple sclerosis.

4. The use according to claim 1 wherein the antibody selectively binds to the polypeptide of SEQ ID NO: 2.

5. The use according to claim 4 wherein the antibody comprises a VH domain comprising an amino acid sequence of SEQ ID NO: 3 and a VL domain comprising an amino acid sequence of SEQ ID NO: 4.

6. The use according to claim 4 wherein the antibody comprises a VH domain comprising an amino acid sequence of SEQ ID NO: 3 and a V_{L} domain comprising an amino acid sequence of SEQ ID NO: 4, internally named Ab1 and competes with an antibody selected form: Monoclonal Anti-human IL-18Rα clone 70614, monoclonal Anti-human IL-18Rα clone 70625, monoclonal anti-human IL-18Rα clone B-E43 and/or monoclonal anti-human IL-18Rα clone H44 for binding to human IL-18Rα.

7. The use according to any one of claims 1 to 6 wherein the antibody against IL-18Rα is to be administered in conjunction with a second therapeutic agent for treating or preventing MS.

8. The use according to claim 7 wherein the antibody against IL-18Rα is to be administered in conjunction with corticosteroïds, immunosuppressive drugs, neuro-protective agents, immunomodulatory drugs or interferons.

9. The use according to claim 8 wherein the antibody against IL-18Rα is to be administered in conjunction with interferon-beta, preferably with interferon beta-1a.

10. A product comprising an antibody against IL-18Rα wherein the antibody has an antigen binding domain that comprises six CDR(s) having an amino acid sequence consisting of: SEQ ID NO: 5 representing CDR1 Heavy chain, SEQ ID NO: 6 representing CDR2 Heavy chain, SEQ ID NO: 7 representing CDR3 Heavy chain, SEQ ID NO: 8 representing CDR1 light chain, SEQ ID NO: 9 representing CDR2 light chain and SEQ ID NO: 10 representing CDR3 light chain and a corticosteroid, an immunosuppressive drug, a neuro-protective agent, an immunomodulatory drug or an interferon as a combined preparation for simultaneous, separate or sequential use in the therapy of Multiple Sclerosis in a mammalian subject, preferably a human subject.

11. The product according to claim 10 wherein the interferon is interferon-beta, preferably interferon beta-1a.

## Patentansprüche

1. Verwendung eines Antikörpers gegen IL-18Rα, wobei der Antikörper eine Antigen bindende Domäne aufweist, welche sechs CDRs mit einer Aminosäuresequenz bestehend aus SEQ ID NO: 5, welche die CDR1 der schweren Kette darstellt, SEQ ID NO: 6, welche die CDR2 der schweren Kette darstellt, SEQ ID NO: 7, welche die CDR3 der schweren Kette darstellt, SEQ ID NO: 8, welche die CDR1 der leichten Kette darstellt, SEQ ID NO: 9, welche die CDR2 der leichten Kette darstellt, und SEQ ID NO: 10, welche die CDR3 der leichten Kette darstellt, umfasst, in der Herstellung eines Medikaments zur Behandlung einer demyelinisierenden Krankheit.

2. Verwendung gemäß Anspruch 1, wobei es sich bei besagter demyelinisierender Krankheit um multiple Sklerose handelt.

3. Verwendung gemäß den Ansprüchen 1 oder 2, wobei der Proband von einer schubförmig remittierenden (RR) multiplen Sklerose, einer sekundär progredienten (SP) multiplen Sklerose, einer primär progredienten (PP) multiplen Sklerose oder einer progredienten schubförmigen (PR) multiplen Sklerose betroffen ist.

4. Verwendung gemäß Anspruch 1, wobei der Antikörper selektiv an das Polypeptid von SEQ ID NO: 2 bindet.

5. Verwendung gemäß Anspruch 4, wobei der Antikörper eine VH-Domäne, welche eine Aminosäuresequenz von SEQ ID NO: 3 umfasst, und eine VL-Domäne, welche eine Aminosäuresequenz von SEQ ID NO: 4 umfasst, enthält.

6. Verwendung gemäß Anspruch 4, wobei der Antikörper eine VH-Domäne, welche eine Aminosäuresequenz von SEQ ID NO: 3 umfasst, und eine V_{L}-Domäne, welche eine Aminosäuresequenz von SEQ ID NO: 4 umfasst, enthält, intern als Ab1 bezeichnet, und mit einem Antikörper, der aus monoklonalem anti-humanem IL-18Rα-Klon 70614, monoklonalem anti-humanem IL-18Rα-Klon 70625, monoklonalem anti-humanem IL-18Rα-Klon B-E43 und/oder monoklonalem anti-humanem IL-18Rα-Klon H44 ausgewählt ist, um die Bindung an humanes IL-18Rα konkurriert.

7. Verwendung gemäß einem der Ansprüche 1 bis 6, wobei der Antikörper gegen IL-18Rα in Verbindung mit einem zweiten therapeutischen Wirkstoff zum Behandeln oder Verhindern von MS zu verabreichen ist.

8. Verwendung gemäß Anspruch 7, wobei der Antikörper gegen IL-18Rα in Verbindung mit Corticosteroiden, immunsuppressiven Arzneimitteln, neuroprotektiven Wirkstoffen, immunmodulatorischen Arzneimitteln oder Interferonen zu verabreichen ist.

9. Verwendung gemäß Anspruch 8, wobei der Antikörper gegen IL-18Rα in Verbindung mit Interferon beta, vorzugsweise mit Interferon beta-1a zu verabreichen ist.

10. Produkt, umfassend einen Antikörper gegen IL-18Rα, wobei der Antikörper eine Antigen bindende Domäne aufweist, welche sechs CDRs mit einer Aminosäuresequenz bestehend aus SEQ ID NO: 5, welche die CDR1 der schweren Kette darstellt, SEQ ID NO: 6, welche die CDR2 der schweren Kette darstellt, SEQ ID NO: 7, welche die CDR3 der schweren Kette darstellt, SEQ ID NO: 8, welche die CDR1 der leichten Kette darstellt, SEQ ID NO: 9, welche die CDR2 der leichten Kette darstellt, und SEQ ID NO: 10, welche die CDR3 der leichten Kette darstellt, umfasst, und ein Corticosteroid, ein immunsuppressives Arzneimittel, einen neuroprotektiven Wirkstoff, ein immunmodulatorisches Arzneimittel oder ein Interferon als Kombinationspräparat, zum simultanen, getrennten oder aufeinanderfolgenden Einsatz in der Behandlung von multipler Sklerose bei einem Probanden, bei dem es sich um einen Säuger handelt, vorzugweise bei einem Probanden, bei dem es sich um einen Menschen handelt.

11. Produkt gemäß Anspruch 10, wobei es sich bei dem Interferon um Interferon beta, vorzugsweise um Interferon beta-1a handelt.

## Revendications

1. Utilisation d'un anticorps dirigé contre l'IL-18Rα, dans laquelle l'anticorps possède un domaine de liaison à un antigène qui comprend six CDR ayant une séquence d'acides aminés consistant en : SÉQ ID NO: 5 représentant une chaîne lourde CDR1, SÉQ ID NO: 6 représentant une chaîne lourde CDR2, SÉQ ID NO: 7 représentant une chaîne lourde CDR3, SÉQ ID NO: 8 représentant une chaîne légère CDR1, SÉQ ID NO: 9 représentant une chaîne légère CDR2 et SÉQ ID NO: 10 représentant une chaîne légère CDR3, dans la fabrication d'un médicament pour le traitement d'une maladie démyélinisante.

2. Utilisation selon la revendication 1, dans laquelle ladite maladie démyélinisante est la sclérose en plaques.

3. Utilisation selon les revendications 1 ou 2, dans laquelle le sujet est affecté par une sclérose en plaques récurrente-rémittente (RR), une sclérose en plaques secondaire progressive (SP), une sclérose en plaques primaire progressive (PP) ou une sclérose en plaques progressive récurrente (PR).

4. Utilisation selon la revendication 1, dans laquelle l'anticorps se lie sélectivement au polypeptide de SÉQ ID NO: 2.

5. Utilisation selon la revendication 4, dans laquelle l'anticorps comprend un domaine VH comprenant une séquence d'acides aminés de SÉQ ID NO: 3 et un domaine VL comprenant une séquence d'acides aminés de SÉQ ID NO: 4.

6. Utilisation selon la revendication 4, dans laquelle l'anticorps comprend un domaine VH comprenant une séquence d'acides aminés de SÉQ ID NO: 3 et un domaine V_{L} comprenant une séquence d'acides aminés de SÉQ ID NO: 4, intrinsèquement appelé Ab1, et est en compétition avec un anticorps sélectionné parmi : le clone 70614 monoclonal anti-IL-18Rα humain, le clone 70625 monoclonal anti-IL-18Rα humain, le clone B-E43 monoclonal anti-IL-18Rα humain et/ou le clone H44 monoclonal anti-IL-18Rα humain, pour une liaison à l'IL-18Rα humain.

7. Utilisation selon l'une quelconque des revendications 1 à 6, dans laquelle l'anticorps dirigé contre l'IL-18Rα est destiné à être administré en conjonction avec un second agent thérapeutique afin de traiter ou de prévenir la SEP.

8. Utilisation selon la revendication 7, dans laquelle l'anticorps dirigé contre l'IL-18Rα est destiné à être administré en conjonction avec des corticostéroïdes, des médicaments immunosuppresseurs, des agents neuroprotecteurs, des médicaments immunomodulateurs ou des interférons.

9. Utilisation selon la revendication 8, dans laquelle l'anticorps dirigé contre l'IL-18Rα est destiné à être administré en conjonction avec l'interféron-bêta, de préférence avec l'interféron bêta-la.

10. Produit comprenant un anticorps dirigé contre l'IL-18Rα, dans lequel l'anticorps possède un domaine de liaison à un antigène qui comprend six CDR ayant une séquence d'acides aminés consistant en SÉQ ID NO: 5 représentant une chaîne lourde CDR1, SÉQ ID NO: 6 représentant une chaîne lourde CDR2, SÉQ ID NO: 7 représentant une chaîne lourde CDR3, SÉQ ID NO: 8 représentant une chaîne légère CDR1, SÉQ ID NO: 9 représentant une chaîne légère CDR2 et SÉQ ID NO: 10 représentant une chaîne légère CDR3, et un corticostéroïde, un médicament immunosuppresseur, un agent neuroprotecteur, un médicament immunomodulateur ou un interféron, en tant que préparation combinée pour une utilisation simultanée, séparée ou séquentielle dans la thérapie de la sclérose en plaques chez un sujet mammalien, de préférence un sujet humain.

11. Produit selon la revendication 10, dans lequel l'interféron est l'interféron bêta, de préférence l'interféron bêta-la.
